# EUROPEAN PATENT SPECIFICATION

(11) **EP 0 719 251 B1**
(45) Date of publication and mention of the grant of the patent: **09.12.1998**
(21) Application number: 94922317.6
(22) Date of filing: 09.08.1994
(51) Int. Cl.: C07C 237/22, C07D 333/24, C07D 257/04, C07D 209/16, C07D 333/20, A61K 31/16, A61K 31/38, A61K 31/41, A61K 31/40

(54) **BICYCLO (2.2.2) OCTANE DERIVATIVES CHOLECYSTOKININ AND/OR GASTRIN ANTAGONISTS**
BICYCLO (2.2.2) OKTAN-DERIVATE MIT CHOLECYSTOKININ UND/ODER GASTRIN ANTAGONISTISCHER WIRKUNG
DERIVES DE BICYCLO 2.2.2 OCTANES UTILISES COMME ANTAGONISTES DE LA CHOLECYSTOKININE ET/OU DE LA GASTRINE

(30) Priority: 12.08.1993 GB 9316722
(43) Date of publication of application: 03.07.1996
(73) Proprietor: JAMES BLACK FOUNDATION LIMITED, London SE24 9JE (GB)
(72) Inventor: KALINDJIAN, Sarkis, Barret, Banstead Surrey SM7 2EJ (GB); STEEL, Katherine, Isobel, Mary, Beckenham Kent BR3 1QP (GB); TOZER, Matthew, John, Upper Norwood London SE19 1TP (GB); HUDSON, Martin, Lyn, Brighton BN1 3JF (GB)
(74) Representative: Fisher, Adrian John
(86) International application number: GB9401740
(87) International publication number: WO9505359

(56) References cited:
- EP-A- 0 405 537
- EP-A- 0 538 945
- WO-A-93/16982

## Description

This invention relates to bicyclo[2.2.2]octane derivatives, and more particularly to bicyclo[2.2.2]octane derivatives which bind to cholecystokinin and/or gastrin receptors. The invention also relates to methods for preparing such bicyclo[2.2.2]octane derivatives.

Gastrin and the CCK's are structurally-related neuropeptides which exist in gastrointestinal tissue and in the CNS (see Mutt V., Gastrointestinal Hormones, Glass G.B.J., ed., Raven Press, N.Y., p 169 and Nisson G., ibid, p. 127).

Gastrin is one of the three primary stimulants of gastric acid secretion. Several forms of gastrin are found including 34-, 17-, and 14-amino acid species with the minimum active fragment being the C-terminal tetrapeptide (TrpMetAspPhe-NH₂) which is reported in the literature to have full pharmacological activity (see Tracey H.J. and Gregory R.A., Nature (London), 1964, 204, 935). Much effort has been devoted to the synthesis of analogues of this tetrapeptide (and the N-protected derivative Boc-TrpMetAspPhe-NH₂) in an attempt to elucidate the relationship between structure and activity.

Natural cholecystokinin is a 33 amino acid peptide (CCK-33), the C-terminal 5 amino acids of which are identical to those of gastrin. Also found naturally is the C-terminal octapeptide (CCK-8) of CCK-33.

The cholecystokinins are reported to be important in the regulation of appetite. They stimulate intestinal motility, gall bladder contraction, pancreatic enzyme secretion, and are known to have a trophic action on the pancreas. They also inhibit gastric emptying and have various effects in the CNS.

Compounds which bind to cholecystokinin and/or gastrin receptors are important because of their potential pharmaceutical use as antagonists of the natural peptides.

A number of gastrin antagonists have been proposed for various therapeutic applications, including the prevention of gastrin-related disorders, gastrointestinal ulcers, Zollinger-Ellison syndrome, antral G Cell hyperplasia and other conditions in which lowered gastrin activity is desirable. The hormone has also been shown to have a trophic action on cells and so an antagonist may be expected to be useful in the treatment of cancers, particularly in the stomach and the colon.

Possible therapeutic uses for cholecystokinin antagonists include the control of appetite disorders such as anorexia nervosa, and the treatment of pancreatic inflammation, biliary tract disease and various psychiatric disorders. Other possible uses are in the potentiation of opiate (e.g. morphine) analgesia, and in the treatment of cancers, especially of the pancreas. Moreover, ligands for cholecystokinin receptors in the brain (so-called CCK_{B} receptors) have been claimed to possess anxiolytic activity.

Our earlier co-pending application WO 9316982 discloses a class of bicyclooctane derivatives which are ligands at gastrin and/or CCK receptors. Included within this class are compounds of the formula 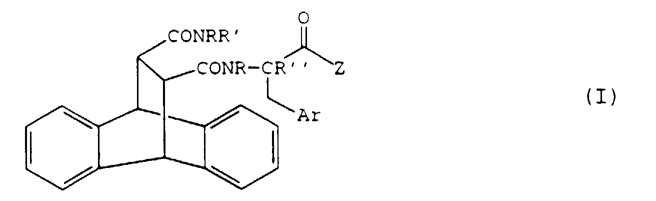 (and ring-substituted derivatives thereof) wherein
each
R group is independently H or C₁ to C₃ alkyl
R' is C₁ to C₁₅ hydrocarbyl (or a derivative thereof),
R'' is H or methyl,
Ar is an aromatic group, and
Z is a substituted amino group.

It has now been found that compounds of especially high activity are obtained when Z is a group of the formula 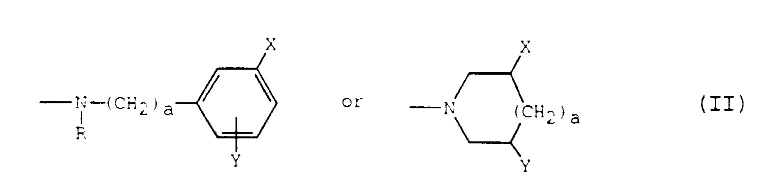 wherein
R is H or C₁ to C₃ alkyl,
X is -CO₂H or tetrazolyl,
Y is H, -CO₂H, tetrazolyl, -CH₂OH, -CO₂Me or -CONH₂, and
a is from 0 to 2.

According to the present invention, therefore, there are provided compounds of the formula 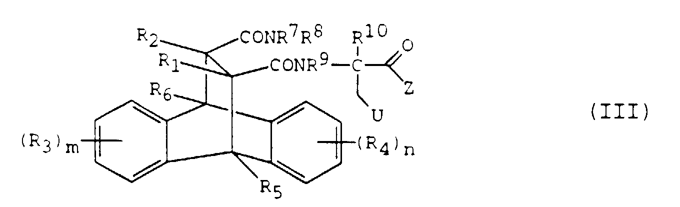 wherein
R¹ and R² are independently H, methyl, halo, carboxy, esterified carboxy, amidated carboxy, carboxymethyl, esterified carboxymethyl or amidated carboxymethyl,
R³ and R⁴ (or each R³ and R⁴ group, when m or n is 2 or more) are independently selected from halo, amino, nitro, cyano, sulphamoyl, sulphonyl, trifluoromethyl, C₁ to C₃ alkyl, C₁ to C₃ alkoxy, hydroxyl, C₁ to C₃ hydroxyalkyl, C₁ to C₃ alkylcarboxyamino, carboxy, esterified carboxy and amidated carboxy
R⁵ and R⁶ are independently selected from H and the groups recited above for R³
m is from 0 to 4, provided that m is not more than 2 unless R³ is exclusively halo,
n is from 0 to 4, provided that n is not more than 2 unless R⁴ is exclusively halo,
R⁷, R⁹ and R¹⁰ are independently H or C₁ to C₃ alkyl,
R⁸ is H or C₁ to C₁₅ hydrocarbyl, in which one or more hydrogen atoms of the hydrocarbyl group may be replaced by a halogen atom, and up to two of the carbon atoms may be replaced by a nitrogen, oxygen or sulphur atom, provided that R⁸ does not contain a -O-O-group,
U is aryl, substituted aryl, heterocyclic (including both saturated and unsaturated groups), substituted heterocyclic or cycloalkyl, and
Z is a group having the formula (II) defined above,
and pharmaceutically acceptable salts thereof.

The invention also comprehends derivative compounds ("pro-drugs") which are degraded *in vivo* to yield the species of formula (III). Pro-drugs are usually (but not always) of lower potency at the target receptor than the species to which they are degraded. Pro-drugs are particularly useful when the desired species has chemical or physical properties which make its administration difficult or inefficient. For example, the desired species may be only poorly soluble, it may be poorly transported across the mucosal epithelium, or it may have an undesirably short plasma half-life. Further discussion of pro-drugs may be found in Stella, V. J. et al, "Prodrugs", Drug Delivery Systems, pp. 112-176 (1985), and Drugs, 29, pp.455-473 (1985).

Pro-drug forms of the pharmacologically-active compounds of the invention will generally be compounds according to formula (III) in which X and/or Y represents an esterified or amidated acid group. Included in such esterified acid groups are groups of the form -COOR¹¹, wherein R¹¹ is C₁ to C₅ alkyl, phenyl, substituted phenyl, benzyl, substituted benzyl, heteroaryl or one of the following: 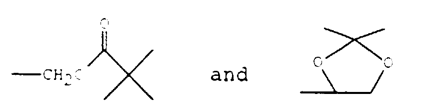 Amidated acid groups include groups of the formula -CONR¹²R¹³, wherein R¹² is H, C₁ to C₅ alkyl, phenyl, substituted phenyl, benzyl, or substituted benzyl, and R¹³ is -OH or one of the groups just recited for R¹².

The term "hydrocarbyl", as used herein, refers to monovalent groups consisting of carbon and hydrogen. Hydrocarbyl groups thus include alkyl, alkenyl, and alkynyl groups (in both straight and branched chain forms), cycloalkyl (including polycycloalkyl), cycloalkenyl, and aryl groups, and combinations of the foregoing, such as alkylaryl, alkenylaryl, alkynylaryl, cycloalkylaryl, and cycloalkenylaryl groups,

A "carbocyclic" group, as the term is used herein, comprises one or more closed chains or rings, which consist entirely of carbon atoms. Included in such groups are alicyclic groups (such as cyclopropyl, cyclobutyl, cyclopentyl, cyclohexyl and adamantyl), groups containing both alkyl and cycloalkyl moieties (such as adamantanemethyl), and aromatic groups (such as phenyl, naphthyl, indanyl, fluorenyl, (1,2,3,4)-tetrahydronaphthyl, indenyl and isoindenyl).

The term "aryl" is used herein to refer to aromatic carbocyclic groups, including those mentioned above.

A "heterocyclic" group comprises one or more closed chains or rings which have at least one atom other than carbon in the closed chain or ring. Examples include benzimidazolyl, thienyl, furanyl, pyrrolyl, imidazolyl, pyrazolyl, thiazolyl, isothiazolyl, oxazolyl, pyrrolidinyl, pyrrolinyl, imidazolidinyl, imidazolinyl, pyrazolidinyl, tetrahydrofuranyl, pyranyl, pyronyl, pyridyl, pyrazinyl, pyridazinyl, piperidyl, piperazinyl, morpholinyl, thionaphthyl, benzofuranyl, isobenzofuryl, indolyl, oxyindolyl, isoindolyl, indazolyl, indolinyl, 7-azaindolyl, isoindazolyl, benzopyranyl, coumarinyl, isocoumarinyl, quinolyl, isoquinolyl, naphthridinyl, cinnolinyl, quinazolinyl, pyridopyridyl, benzoxazinyl, quinoxadinyl, chromenyl, chromanyl, isochromanyl and carbolinyl.

The term "halogen", as used herein, refers to any of fluorine, chlorine, bromine and iodine. Most usually, however, halogen substituents in the compounds of the invention are chlorine or fluorine substituents.

Preferably, m and n are both 0. However, when m and n are not both 0, R³ and R⁴ are preferably selected from halo, hydroxy, amino, nitro, cyano, sulphamoyl, C₁ to C₃ alkyl and C₁ to C₃ alkoxy. As mentioned above, when m or n is 2 or more, each R³ and R⁴ group is independent of the others. For example, the compounds of the invention may include two different R³ groups.

Particularly preferred groups for R⁵ and R⁶ are hydrogen and the groups just recited for R³, and especially hydrogen, methyl and fluoro.

When reference is made herein to a "substituted" aromatic group, the substituents will generally be from 1 to 3 in number (and more usually 1 or 2 in number), and generally selected from the groups recited above for R³. However, halo substituents may be up to 5 in number.

Preferably, R⁸ is C₆ to C₈ straight or branched chain alkyl or cycloalkyl, or R¹¹-(CH₂)ₚ-, wherein R¹¹ is selected from phenyl, 1-naphthyl, 2-naphthyl, indolyl, norbornyl, 1-adamantyl, 2-adamantyl, cyclohexyl or cycloheptyl, and p is from 0 to 3.

Pharmaceutically acceptable salts of the acidic compounds of the invention include salts with alkali metals and alkaline earth metals, such as sodium, potassium, calcium and magnesium, and salts with organic bases. Suitable organic bases include amines such as N-methyl-D-glucamine.

Pharmaceutically acceptable salts of the basic compounds of the invention include salts derived from organic or inorganic acids. Suitable acids include hydrochloric acid, phosphoric acid, oxalic acid, maleic acid, succinic acid and citric acid.

The compounds of the invention exist in various enantiomeric and diastereomeric forms. It will be understood that the invention comprehends the different enantiomers and diastereomers in isolation from each other, as well as mixtures of enantiomers and diastereomers. Also, the structural formulae herein show the groups R¹ and R² arranged cis to each other, but it will be appreciated that the invention includes the corresponding trans isomers.

Compounds according to the present invention may conveniently be made by the process depicted in Reaction Scheme A.

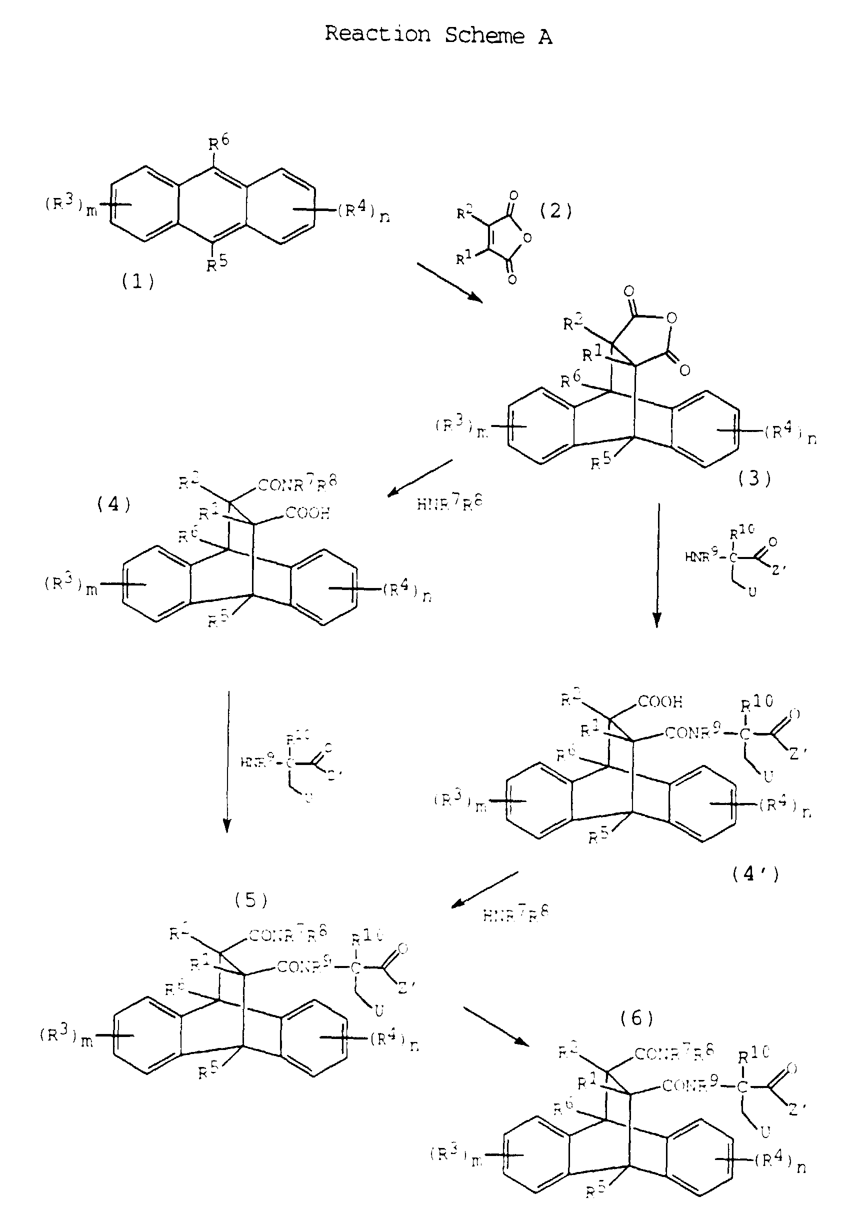

In this scheme, anthracene or an anthracene derivative (1) is reacted with the acid anhydride (2) in a Diels-Alder reaction. The reactants are conveniently refluxed together in a suitable solvent such as toluene to form the adduct (3). In some cases, it may be appropriate to conduct the reaction at elevated pressure and/or in the presence of a Lewis acid catalyst.

The adduct (3) is then reacted with an amine of formula HNR⁷R⁸ to form the acid compound (4). The reaction is suitably carried out in a solvent such as THF in the presence of a base such as DMAP.

The acid compound (4) is then reacted with a compound of formula 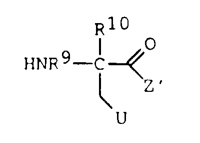 in which Z' represents a suitably protected form of the group Z, to form protected intermediate (5). Deprotection of Z' (eg. by hydrogenation over a palladium catalyst) then yields the desired compound (6).

Alternatively, as shown in Reaction Scheme A, the two amidation reactions may be carried out in the reverse order, proceeding via the intermediate (4').

Suitable amidation methods are described in detail in "The Peptides, Vol. 1", Gross and Meinenhofer, Eds., Academic Press, N.Y., 1979. These include the carbodiimide method (using, for example, 1,3-dicyclohexylcarbodiimide [DCC] or 1-(3-dimethylaminopropyl)-3-ethylcarbodiimide hydrochloride [EDCI], and optionally an additive such as 1-hydroxybenzotriazole [HOBT] to prevent racemization), the azide method, the mixed anhydride method, the symmetrical anhydride method, the acid chloride method, the use of bis (2-oxo-3-oxazolidinyl) phosphinic chloride [BOP-Cl], the use of PyBOP or PyBrOP, the use of the isopropenylsuccinimido carbonate method and the active ester method (using, for example, N-hydroxysuccinimide esters, 4-nitrophenyl esters or 2,4,5-trichlorophenol esters).

The coupling reactions are generally conducted under an inert atmosphere, such as an atmosphere of nitrogen or argon. Suitable solvents for the reactants include methylene chloride, tetrahydrofuran [THF], dimethoxyethane [DME] and dimethylformamide [DMF].

The equivalent trans adducts can be prepared using a suitably differentiated fumaric acid (eg the mono methyl mono benzyl diester), which, after addition to anthracene or an anthracene derivative (1), allows independent elaboration of the two sidechains.

The invention therefore also provides a method of making a compound according to formula (III) above, said method including the step of reacting a compound of the formula 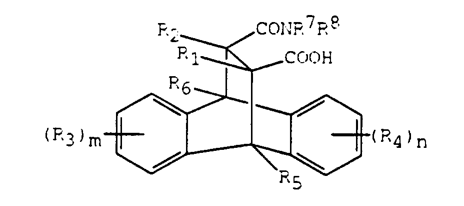 with a suitably protected compound of formula

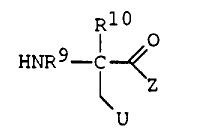

As already mentioned, it may be desired to carry out the amidation reactions in a different order. The invention therefore also comprehends a method of making a compound according to formula (III) above, said method including the step of reacting a compound of the formula

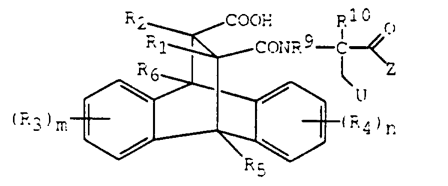

with a suitably protected compound of formula HNR⁷R⁸, or the step of reacting a compound of the formula 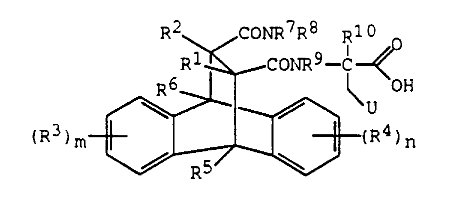 with a suitably protected compound of formula 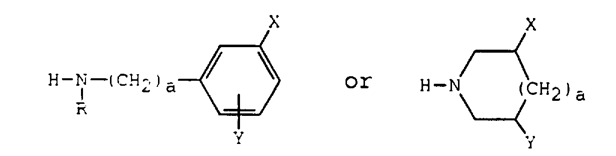 Pharmaceutically acceptable salts of the acidic or basic compounds of the invention can of course be made by conventional procedures, such as by reacting the free base or acid with at least a stoichiometric amount of the desired salt-forming acid or base.

The compounds of the invention can be administered by oral or parenteral routes, including intravenous, intramuscular, intraperitoneal, subcutaneous, rectal and topical administration.

For oral administration, the compounds of the invention will generally be provided in the form of tablets or capsules or as an aqueous solution or suspension.

Tablets for oral use may include the active ingredient mixed with pharmaceutically acceptable excipients such as inert diluents, disintegrating agents, binding agents, lubricating agents, sweetening agents, flavouring agents, colouring agents and preservatives. Suitable inert diluents include sodium and calcium carbonate, sodium and calcium phosphate, and lactose, while corn starch and alginic acid are suitable disintegrating agents. Binding agents may include starch and gelatin, while the lubricating agent, if present, will generally be magnesium stearate, stearic acid or talc. If desired, the tablets may be coated with a material such as glyceryl monostearate or glyceryl distearate, to delay absorption in the gastrointestinal tract.

Capsules for oral use include hard gelatin capsules in which the active ingredient is mixed with a solid diluent, and soft gelatin capsules wherein the active ingredient is mixed with water or an oil such as peanut oil, liquid paraffin or olive oil.

For intramuscular, intraperitoneal, subcutaneous and intravenous use, the compounds of the invention will generally be provided in sterile aqueous solutions or suspensions, buffered to an appropriate pH and isotonicity. Suitable aqueous vehicles include Ringer's solution and isotonic sodium chloride. Aqueous suspensions according to the invention may include suspending agents such as cellulose derivatives, sodium alginate, polyvinylpyrrolidone and gum tragacanth, and a wetting agent such as lecithin. Suitable preservatives for aqueous suspensions include ethyl and n-propyl p-hydroxybenzoate.

Effective doses of the compounds of the present invention may be ascertained by conventional methods. The specific dosage level required for any particular patient will depend on a number of factors, including the severity of the condition being treated and the weight of the patient. In general, however, the daily dose (whether administered as a single dose or as divided doses) will be in the range 0.001 to 5000 mg per day, more usually from 1 to 1000 mg per day, and most usually from 10 to 200 mg per day. Expressed as dosage per unit body weight, a typical dose will be between 0.01 µg/kg and 50mg/kg, especially between 10 µg/kg and 10 mg/kg, eg. between 100 µg/kg and 2 mg/kg.

The invention is now further illustrated by means of the following examples.

Example 1 Preparation of cis-7- (1S- (3, 5-dicarboxyphenylamino-carbonyl) -2-phenylethylaminocarbonyl)-8-(1-adamantanemethylamino-carbonyl) -2,3,5,6-dibenzobicyclo[2.2.2] octane Diastereoisomer 1
a. 2,3,5,6-dibenzobicyclo[2.2.2]octane-7,8-dicarboxylic acid anhydride
   Anthracene (8.9 g, 0.05 mol) and maleic anhydride (4.9 g, 0.05 mol) were refluxed for 3h in toluene (200 ml). Upon cooling the title compound was obtained as white crystals which were isolated by filtration (10.2g, 74%).
   b. (±)-cis-8-(1-adamantanemethylaminocarbonyl)-2,3, 5, 6-dibenzo bicyclo[2.2.2]octane-7-carboxylic acid
      2,3,5,6-dibenzobicyclo(2.2.2.)octane-7,8-dicarboxylic acid anhydride (prepared in step a) (276 mg, 1.0 mmol) and 1-adamantanemethylamine (182 mg, 1.1 mmol) were dissolved in dry THF (5 ml) and refluxed for 1h. A thick white precipitate was formed and this was isolated by filtration and washed with THF to leave the title compound (320 mg, 72%), m.p. 237-9°, found: C,78.76; H, 7.18; N, 3.33. C₂₉H₃₁NO₃ requires C, 78.88; H, 7.08; N, 3.17%
   c. 3,5-dibenzyloxycarbonyl-nitrobenzene
      5-nitro-isophthalic acid (21.lg, 0.1 mol), thionyl chloride (80 ml) and DMF (10 drops) were stirred and heated for about 1h until a clear solution was obtained. Excess thionyl chloride was removed by evaporation and the residual acid chloride was coevaporated with dichloromethane (2 x 100 ml) to remove the last traces.
      Benzyl alcohol (21.6 g, 0.2 mol) and triethylamine (30.03 g, 0.3 mol) were dissolved in dichloromethane (200 ml) and stirred at 0° under an atmosphere of dry nitrogen and a solution of the acid chloride in dichloromethane (50 ml) was added dropwise over 20 min. The solution was stirred and refluxed for 1h, and the solution was cooled. The organic layer was washed with water (2 x 100ml), saturated sodium hydrogencarbonate solution (100 ml) and dried over magnesium sulphate. The solution was filtered and evaporated to leave the title compound (39.1g, 100%).
   d. 3,5-dibenzyloxycarbonyl-aniline
      The nitro compound prepared in step c above (3.91g, 10 mol) was dissolved in ethyl acetate (50 ml) and tin(II)chloride dihydrate (11.27g, 50 mmol) was added and the mixture stirred and heated at 70° under an atmosphere of nitrogen for 1h. The mixture was poured carefully onto 5% sodium hydrogencarbonate solution (200 ml) and a further aliquot of ethyl acetate (100 ml) was added. After shaking the organic layer was separated and the aqueous layer was extracted with more ethyl acetate (50 ml). The combined organic layers were washed with brine, and dried, filtered and evaporated to leave a pale yellow solid (3.25g, 90%).
   e. N-tert-butyloxycarbonyl-1S-(3,5-dibenzyloxycarbonylphenylamino-carbonyl)-2-phenylethylamine
      BOC-L-phenylalanine (8.76 g, 33 mmol) was dissolved in dry dichloromethane (200 ml) and dry diisopropylethylamine (11.48 ml, 66 mmol) was added followed by PyBROP (15.33g, 33 mmol). The mixture was stirred at room temperature for 5 min and then the amine prepared in step d above (7.22 g, 20 mmol) was added. The solution was stirred at room temperature for a further 5h and the solution was then washed sequentially with 2M hydrochloric acid, water, saturated sodium hydrogencarbonate solution and water and finally dried, filtered and evaporated to leave an oil. This was purified by column chromatography (90% dichloromethane and 10% ethyl acetate) to leave the title compound as a white solid (11.0 g, 90%)
   f. cis-7-(1S- (3,5-dibenzyloxycarbonylphenylaminocarbonyl)-2-phenylethylaminocarbonyl) -8- (1-adamantanemethylaminocarbonyl) -2, 3,5,6-dibenzobicyclo[2.2.2]octane Diastereoisomer 1
      N-tert-butyloxycarbonyl-1S-(3,5-dibenzyloxycarbonylphenylaminocarbonyl)-2-phenylethylamine prepared in step e above (8.0 g, 13 mmol) was dissolved in trifluoroacetic acid (40 ml) and stirred at room temperature for 30 min. The solvent was removed by evaporation and the residue taken up in dry dichloromethane (50 ml) and basified with diisopropylethylamine.
      Meanwhile (+) -cis-8-(1-adamantanemethylaminocarbonyl)-2,3,5,6-dibenzo bicyclo[2.2.2]octane-7-carboxylic acid prepared in step b above (5.75 g, 13 mmol) was suspended in dry dichloromethane (150 ml) and diisopropylethylamine (4.6 ml, 26 mmol) was added followed by PyBOP (6.76 g, 13 mmol). The solution was stirred until a clear solution was obtained and the solution of amine prepared above was added. After stirring at room temperature for 3h, the solution was washed sequentially with 2M hydrochloric acid and water, dried, filtered and evaporated. The residual oil was purified by column chromatography (90% dichloromethane and 10% ethyl acetate) to leave two compounds. The less polar material was designated the title compound (4.65 g).
   g. cis-7-(1S-(3,5-dicarboxyphenylaminocarbonyl)-2-phenylethylaminocarbonyl)-8-(1-adamantanemethylaminocarbonyl)-2,3,5,6-dibenzobicyclo[2.2.2]octane Diastereoisomer 1
      The dibenzylester prepared in step f above (4.65g, 5.0 mmol) was dissolved in 1:1 methanolic THF (40 ml). 10% palladium on charcoal (400 mg) was added and the reaction was stirred under an atmosphere of hydrogen overnight. The catalyst was removed by filtration through celite and the product isolated by evaporation (3.53 g).
      The compound was further characterised and tested as the di-N-methyl-D-glucamine salt found: C, 61.38; H, 7.54; N, 4.24. C₆₀H₇₉N₅O₁₇. 4.2 mol dioxan requires C, 60.99; H, 7.51; N, 4.63%

Example 2 Preparation of cis-7-(1S-(3,5-dicarboxyphenylaminocarbonyl) -2-phenylethylaminocarbonyl)-8-(1-adamantanemethylaminocarbonyl)-2,3,5,6-dibenzobicyclo[2.2.2] octane Diastereoisomer 2

The compound was prepared essentially as in example 1 except that the more polar material from the chromatography described in step f was used in the final hydrogenation.

The compound was further characterised and tested as the N-methyl-D-glucamine salt found: C, 64.34; H, 6.64; N, 4.70. C₅₃H₆₂N₄O₁₂. 1.7 mol dioxan. 0.8 H₂O requires C, 64.63; H, 7.00; N, 5.04%

Example 3 Preparation of cis-7-(1R- (3,5-dicarboxyphenylaminocarbonyl) -2-phenylethylaminocarbonyl)-8-(1-adamantanemethylaminocarbonyl) -2,3,5,6-dibenzobicyclo[2.2.2]octane Diastereoisomer 1

The compound was prepared essentially as in example 1 except that BOC-D-phenylalanine was used in step e instead of BOC-L-phenylalanine.

The compound was further characterised and tested as the N-methyl-D-glucamine salt found: C, 65.66; H, 6.68; N, 5.46. C₅₃H₆₂N₄O₁₂. 1.3 H₂O requires C, 65.55; H, 6.71; N, 5.76%

Example 4 Preparation of cis-7-(1R-(3,5-dicarboxyphenylaminocarbonyl)-2-phenylethylaminocarbonyl)-8-(1-adamantananethylaminocarbonyl) -2,3,5,6-dibenzobicyclo[2.2.2]octane Diastereoisomer 2

The compound was prepared essentially as in example 3 except that the more polar material from the chromatography described in step f was used in the final hydrogenation.

The compound was further characterised and tested as the N-methyl-D-glucamine salt found: C, 64.77; H, 7.00; N, 5.90. C₅₃H₆₂N₄O₁₂. 2.0 H₂O requires C, 64.75; H, 6.76; N, 5.69%

Example 5 Preparation of cis-7-(1S-(3,5-dicarboxyphenylaminocarbonyl) -2-cyclohexylethylaminocarbonyl)-8-(1-adamantanemethylaminocarbonyl) -2,3,5,6- dibenzobicyclo[2.2.2]octane (mixture of diastereomers)

The compound was prepared essentially as in example 1 except that BOC-L-2-cyclohexylalanine was used in step e instead of BOC-L-phenylalanine and no attempt was made to separate the diastereomers in step.

The compound was further characterised and tested as the di -N-methyl-D-glucamine salt found: C, 60.31; H, 8.00; N, 5.84. C₆₀H₈₅N₅O₁₇. 2.5 H₂O requires C, 60.38; H, 7.60; N, 5.86%

Example 6 Preparation of cis-7-(1S-(3,5-dicarboxyphenylaminocarbonyl) -2-(2-thiophenyl)-ethylaminocarbonyl) -8-(1-adamantane-methylaminocarbonyl) -2,3,5,6-dibenzobicyclo[2.2.2]octane Diastereoisomer 1

The compound was prepared essentially as in example 1 except that BOC-L-2-thiophenylalanine was used in step e instead of BOC-L-phenylalanine.

The compound was further characterised and tested as the N-methyl-D-glucamine salt found: C, 58.61; H, 6.43; N, 5.00. C₅₁H₆₀N₄0₁₂S. 5.0 H₂O requires C, 58.72; H, 6.76; N, 5.37%

Example 7 Preparation of cis-7-(1S-(3,5-dicarboxyphenylaminocarbonyl) -2- (2-thiophenyl) ethylaminocarbonyl)-8-(1-adamantanemethylaminocarbonyl) -2,3,5,6-dibenzobicyclo[2.2.2]octane Diastereoisomer 2

The compound was prepared essentially as in example 6 except that the more polar material from the chromatography described in step f was used in the final hydrogenation.

The compound was further characterised and tested as the N-methyl-D-glucamine salt found: C, 59.26; H, 6.66; N, 5.00. C₅₁H₆₀N₄O₁₂S. 4.5 H₂O requires C, 59.23; H, 6.73; N, 5.40%

Example 8 Preparation of cis-7-(1S-(3,5-dicarboxyphenylaminocarbonyl) -2-(4-fluorophenyl)-ethylaminocarbonyl)-8-(1-adamantane-methylaminocarbonyl)-2,3,5,6-dibenzobicyclo[2.2.2]octane Diastereoisomer 1

The compound was prepared essentially as in example 1 except that BOC-L-4-fluorophenylalanine was used in step e instead of BOC-L-phenylalanine.

The compound was further characterised and tested as the di-N-methyl-D-glucamine salt found: C, 59.38; H, 7.19; N, 5.92. C₆₀H₇₈N₅O₁₇F. 3.1 H₂O requires C, 59.24; H, 6.98; N, 5.76%

Example 9 Preparation of cis-7-(1S-(3,5-dicarboxyphenylaminocarbonyl) -2-(4-fluorophenyl)ethylaminocarbonyl)-8-(1-adamantanemethylaminocarbonyl) -2,3,5,6-dibenzobicyclo[2.2.2]octane Diastereoisomer 2

The compound was prepared essentially as in example 8 except that the more polar material from the chromatography described in step f was used in the final hydrogenation.

The compound was further characterised and tested as the di-N-methyl-D-glucamine salt found: C, 60.18; H, 7.21; N, 6.01. C₆₀H₇₈N₅O₁₇ F. 2.3 H₂O requires C, 59.94; H, 6.93; N, 5.83%

Example 10 Preparation of cis-7-(1S-(3,5-dicarboxyphenylaminocarbonyl)-2-(4-chlorophenyl)-ethylaminocarbonyl)-8-(1-adamantanemethylaminocarbonyl) -2,3,5,6-dibenzobicyclo[2.2.2]octane Diastereoisomer 1

The compound was prepared essentially as in example 1 except that BOC-L-4-chlorophenylalanine was used in step e instead of BOC-L-phenylalanine.

The compound was further characterised and tested as the di-N-methyl-D-glucamine salt found: C, 59.16; H, 7.04; N, 5.72. C₆₀H₇₈N₅O₁₇Cl. 1.9 H₂O requires C, 59.49; H, 6.81; N, 5.78%

Example 11 Preparation of cis-7-(1S-(3,5-dicarboxyphenylaminocarbonyl)-2-(4-chlorophenyl)ethylaminocarbonyl)-8-(1-adamantanemethylaminocarbonyl) -2,3,5,6-dibenzobicyclo[2.2.2]octane Diastereoisomer 2

The compound was prepared essentially as in example 10 except that the more polar material from the chromatography described in step f was used in the final hydrogenation.

The compound was further characterised and tested as the di-N-methyl-D-glucamine salt found: C, 59.08; H, 6.97; N, 5.43. C₆₀H₇₈N₅O₁₇Cl. 2.7 H₂O requires C, 58.85; H, 6.86; N, 5.72%

Example 12 Preparation of cis-7-(1S-(3,5-dicarboxyphenylaminocarbonyl)-2-(4-methoxyphenyl) -ethylaminocarbonyl)-8-(1-adamantanemethylaminocarbonyl) -2,3,5,6-dibenzobicyclo[2.2.2]octane Diastereoisomer 1

The compound was prepared essentially as in example 1 except that BOC-L-4-methoxyphenylalanine was used in step e instead of BOC-L-phenylalanine.

The compound was further characterised and tested as the di-N-methyl-D-glucamine salt found: C, 59.36; H, 7.28; N, 5.77. C₆₁H₈₁N₅O₁₈. 3.3 H₂O requires C, 59.45; H, 7.17; N, 5.68%

Example 13 Preparation of cis-7-(1S-(3,5-dicarboxyphenylaminocarbonyl) -2- (4-methoxyphenyl)ethylaminocarbonyl)-8-(1-adamantanemethylaminocarbonyl) -2,3,5,6-dibenzobicyclo[2.2.2]octane Diastereoisomer 2

The compound was prepared essentially as in example 12 except that the more polar material from the chromatography described in step f was used in the final hydrogenation.

The compound was further characterised and tested as the di-N-methyl-D-glucamine salt found: C, 57.14; H, 7.26; N, 5.32. C₆₁H₈₁N₅O₁₈. 6.3 H₂O requires C, 57.01; H, 7.34; N, 5.45%

Example 14 Preparation of cis-7-(1S-(3,5-dicarboxyphenylaminocarbonyl)-2-(2-naphthyl) -ethylaminocarbonyl)-8-(1-adamantanemethylaminocarbonyl)-2,3,5,6-dibenzobicyclo[2.2.2]octane Diastereoisomer 1

The compound was prepared essentially as in example 1 except that BOC-L-3-(2-naphthyl)alanine was used in step e instead of BOC-L-phenylalanine.

The compound was further characterised and tested as the di-N-methyl-D-glucamine salt found: C, 61.14 H, 7.03; N, 5.64. C₆₄H₈₁N₅O₁₇. 3.5 H₂O requires C, 61.20; H, 7.07; N, 5.58%

Example 15 Preparation of cis-7-(1S-(3,5-dicarboxyphenylaminocarbonyl)-2- (2-naphthyl)ethylaminocarbonyl)-8-(1-adamantanemethylaminocarbonyl) -2,3,5,6-dibenzobicyclo[2.2.2]octane Diastereoisomer 2

The compound was prepared essentially as in example 14 except that the more polar material from the chromatography described in step f was used in the final hydrogenation.

The compound was further characterised and tested as the di-N-methyl-D-glucamine salt found: C, 61.59; H, 7.11; N, 5.52. C₆₄H₈₁N₅O₁₇. 4.2 H₂O requires C, 61.59; H, 7.11; N, 5.52%

Example 16 Preparation of cis-7-(1S-(3,5-dicarboxyphenylaminocarbonyl)-2- (2-fluorophenyl)-ethylaminocarbonyl)-8-(1-adamantanemethylaminocarbonyl) -2,3,5,6-dibenzobicyclo[2.2.2]octane Diastereoisomer 1

The compound was prepared essentially as in example 1 except that BOC-L-2-fluorophenylalanine was used in step e instead of BOC-L-phenylalanine.

The compound was further characterised and tested as the di-N-methyl-D-glucamine salt found: C, 58.63; H, 6.99; N, 5.49. C₆₀H₇₈N₅O₁₇F. 4.0 H₂O requires C, 58.44; H, 7.04; N, 5.68%

Example 17 Preparation of cis-7-(1S-(3,5-dicarboxyphenylaminocarbonyl)-2- (2-fluorophenyl)ethylaminocarbonyl)-8-(1-adamantanemethylaminocarbonyl) -2,3,5,6-dibenzobicyclo[2.2.2]octane Diastereoisomer 2

The compound was prepared essentially as in example 16 except that the more polar material from the chromatography described in step f was used in the final hydrogenation.

The compound was further characterised and tested as the di-N-methyl-D-glucamine salt found: C, 58.49; H, 7.00; N, 5.67. C₆₀H₇₈N₅O₁₇F. 4.2 H₂O requires C, 58.34; H, 7.04; N, 5.67%

Example 18 Preparation of cis-7-(1S-(3,5-dicarboxyphenylaminocarbonyl)-2- (3-fluorophenyl) -ethylaminocarbonyl)-8-(1-adamantanemethylaminocarbonyl)-2,3,5,6 -dibenzobicyclo[2.2.2]octane Diastereoisomer 1

The compound was prepared essentially as in example 1 except that BOC-L-3-fluorophenylalanine was used in step e instead of BOC-L-phenylalanine.

The compound was further characterised and tested as the di-N-methyl-D-glucamine salt found: C, 58.60; H, 7.04; N, 5.62. C₆₀H₇₈N₅O₁₇F. 3.9 H₂O requires C, 58.55; H, 7.038; N, 5.69%

Example 19 Preparation of cis-7-(1S-(3,5-dicarboxyphenylaminocarbonyl)-2- (3-fluorophenyl)ethylaminocarbonyl)-8-(1-adamantanemethylaminocarbonyl) -2,3,5,6-dibenzobicyclo[2.2.2]octane Diastereoisomer 2

The compound was prepared essentially as in example 18 except that the more polar material from the chromatography described in step f was used in the final hydrogenation.

The compound was further characterised and tested as the di-N-methyl-D-glucamine salt found: C, 58.49; H, 7.09; N, 5.72. C₆₀H₇₈N₅O₁₇F. 3.9 H₂O requires C, 58.544; H, 7.03; N, 5.69%

Example 20 Preparation of cis-7-(1S-(3,5-dicarboxyphenylaminocarbonyl)-2- (2-chlorophenyl) -ethylaminocarbonyl)-8-(1-adamantanemethylaminocarbonyl) -2,3,5, 6-dibenzobicyclo [2.2.2] octane (mixture of diastereomers)

The compound was prepared essentially as in example 1 except that BOC-L-2-chlorophenylalanine was used in step e instead of BOC-L-phenylalanine and no attempt was made to separate the diastereomers in step f.

The compound was further characterised and tested as the di-N-methyl-D-glucamine salt found: C, 57.69; H, 7.05; N, 5.55. C₆₀H₇₈N₅O₁₇Cl. 4.1 H₂O requires C, 57.59; H, 6.95; N, 5.55%

Example 21 Preparation of cis-7-(1R-(3,5-dicarboxyphenylaminocarbonyl)-2- (2-thiophenyl)ethylaminocarbonyl)-8-(1-adamantanemethylaminocarbonyl)-2,3,5,6-dibenzobicyclo[2.2.2]octane Diastereoisomer 2

The compound was prepared essentially as in example 7 except that except that BOC-D-2-thiophenylalanine was used in step e instead of BOC-L-phenylalanine.

The compound was further characterised and tested as the di-N-methyl-D-glucamine salt

Example 22 (comparative) Preparation of cis-7-(1S- (phenylaminocarbonyl)-2-phenyl-ethylaminocarbonyl)-8-(1-adamantanemethylaminocarbonyl) -2,3,5,6-dibenzobicyclo[2.2.2]octane Diastereoisomer 1

The compound was prepared essentially as in example 1 step f except that N-tert-butyloxycarbonyl-1S- (phenylaminocarbonyl)-2-phenylethylamine was used instead of N-tert-butyloxycarbonyl-1S-(3,5-dibenzyloxycarbonylphenylaminocarbonyl)-2-phenylethylamine. As before, the less polar compound after chromatography was designated the compound of this example. found: C, 78.33; H, 6.94; N, 6.05 C₄₄H₄₅N₃O₃. 0.4 ethyl acetate requires C,78.49; H, 6.94; N, 6.05%

Example 23 (comparative) Preparation of cis-7-(1S-(phenylaminocarbonyl)-2-phenylethylaminocarbonyl)-8-(1-adamantanemethylaminocarbonyl)-2,3,5,6-dibenzobicyclo[2.2.2]octane Diastereoisomer 2

The compound was prepared essentially as in example 22 except that the more polar material from the chromatography was designated as the compound of this example found: C, 79.37; H, 6.96; N, 6.24 C₄₄H₄₅N₃O₃ requires C,79.61; H, 6.83; N, 6.33%

Example 24 Preparation of cis-7-(1S-(3,5-dicarboxyphenylaminocarbonyl) -2-(4-hydroxyphenyl) -ethylaminocarbonyl)-8-(1-adamantanemethylaminocarbonyl) -2,3,5,6-dibenzobicyclo[2.2.2]octane

### Diastereoisomer 1

The compound was prepared essentially as in example 1 except that BOC-L-tyrosine was used in step e instead of BOC-L-phenylalanine.

The compound was further characterised and tested as the di-N-methyl-D-glucamine salt

Example 25 Preparation of cis-7-(1S-(3,5-dicarboxyphenylaminocarbonyl)-2 -(4-hydroxyphenyl)ethylaminocarbonyl)-8-(1-adaxantanemethylaminocarbonyl)-2,3,5,6 -dibenzobicyclo[2.2.2]octane Diastereoisomer 2

The compound was prepared essentially as in example 24 except that the more polar material from the chromatography described in step f was used in the final hydrogenation.

The compound was further characterised and tested as the di-N-methyl-D-glucamine salt found: C, 57.11; H, 7.46; N, 5.79. C₆₀H₇₉N₅O₁₈. 6.0 H₂O requires C, 56.95; H, 7.24; N, 5.53%

Example 26 Preparation of cis-7-(1R-(3,5-dicarboxyphenylaminocarbonyl)-2-(4-hydroxyphenyl)-ethylaminocarbonyl)-8-(1-adamantanemethylaminocarbonyl) -2,3,5, 6-dibenzobicyclo [2.2.2 ]octane Diastereoisomer 1

The compound was prepared essentially as in example 1 except that BOC-D-tyrosine was used in step e instead of BOC-L-phenylalanine.

The compound was further characterised and tested as the di-N-methyl-D-glucamine salt found: C, 57.05; H, 7.09; N, 5.23. C₆₀H₇₉N₅O₁₈. 6.1 H₂O requires C, 56.79; H, 7.25; N, 5.52%

Example 27 Preparation of cis-7-(1R-(3,5-dicarboxyphenylaminocarbonyl)-2-(4-hydroxyphenyl)ethylaminocarbonyl)-8-(1-adamantanemethylaminocarbonyl)-2,3,5,6-dibenzobicyclo [2.2.2 ]octane Diastereoisomer 2

The compound was prepared essentially as in example 27 except that the more polar material from the chromatography described in step f was used in the final hydrogenation.

Example 28 Preparation of cis-7-(1S-(3,5-dicarboxyphenylaminocarbonyl) -2-phenylethylaminocarbonyl)-8-(1-cycloheptane-methylaminocarbonyl) -2,3,5,6-dibenzobicyclo[2.2.2]octane Diastereoisomer 1

The compound was prepared essentially as in example 1 except that cycloheptanemethylamine was used in step b instead of 1-adamantanemethylamine.

The compound was further characterised and tested as the di-N-methyl-D-glucamine salt found: C, 59.25; H, 7.17; N, 5.94. C₅₇H₇₇N₅O₁₇. 2.9 H₂O requires C, 59.14; H, 7.22; N, 6.06%

Example 29 Preparation of cis-7-(1S-(3,5-dicarboxyphenylaminocarbonyl) -2-phenylethylaminocarbonyl) -8-(cycloheptanemethyl-aminocarbonyl)-2,3,5,6-dibenzobicyclo[2.2.2]octane Diastereoisomer 2

The compound was prepared essentially as in example 28 except that the more polar material from the chromatography described in step f was used in the final hydrogenation.

The compound was further characterised and tested as the di-N-methyl-D-glucamine salt found: C, 59.14; H, 7.15; N, 6.10. C₅₇H₇₇N₅O₁₇. 2.9 H₂O requires C, 59.14; H, 7.22; N, 6.06%

Example 30 Preparation of cis-7-(1S-(3,5-dicarboxyphenylaminocarbonyl)-2-phenylethylaminocarbonyl)-8-(1-cyclohexanemethylaminocarbonyl)-2,3,5,6-dibenzobicyclo [2.2.2] octane Diastereoisomer 1

The compound was prepared essentially as in example 1 except that cyclohexanemethylamine was used in step b instead of 1-adamantanemethylamine.

The compound was further characterised and tested as the di-N-methyl-D-glucamine salt found: C, 58.75; H, 7.11; N, 5.85. C₅₆H₇₅N₅O₁₇. 3.2 H₂O requires C, 58.58; H, 7.15; N, 6.10%

Example 31 Preparation of cis-7-(1S-(3,5-dicarboxyphenylaminocarbonyl) -2-phenylethylaminocarbonyl)-8-(cyclohexanemethylaminocarbonyl) -2,3,5,6-dibenzobicyclo[2.2.2]octane Diastereoisomer 2

The compound was prepared essentially as in example 30 except that the more polar material from the chromatography described in step f was used in the final hydrogenation.

The compound was further characterised and tested as the di-N-methyl-D-glucamine salt found: C, 58.61; H, 7.19; N, 6.18. C₅₆H₇₅N₅O₁₇. 3.1 H₂O requires C, 58.69; H, 7.14; N, 6.11%

Example 32 Preparation of cis-7-(1S-(3,5-dicarboxyphenylaminocarbonyl)-2-phenylethylaminocarbonyl)-8-(1-naphthalenemethylaminocarbonyl) -2,3,5,6-dibenzobicyclo [2.2.2]octane Diastereoisomer 1

The compound was prepared essentially as in example 1 except that 1-naphthalenemethylamine was used in step b instead of 1adamantanemethylamine.

The compound was further characterised and tested as the di-N-methyl-D-glucamine salt found: C, 59.82; H, 6.70; N, 6.04. C₆₀H₇₁N₅O₁₇. 3.7 H₂O requires C, 60.03; H, 6.58; N, 5.83%

Example 33 Preparation of cis-7-(1S-(3,5-dicarboxyphenylaminocarbonyl)-2-phenylethylaminocarbonyl)-8-(1-naphthalenemethylaminocarbonyl)-2,3,5,6-dibenzobicyclo[2.2.2]octane Diastereoisomer 2

The compound was prepared essentially as in example 32 except that the more polar material from the chromatography described in step f was used in the final hydrogenation.

The compound was further characterised and tested as the di-N-methyl-D-glucamine salt found: C, 58.02; H, 6.84; N, 5.96. C₆₀H₇₁N₅O₁₇. 5.6 H₂O requires C, 58.31; H, 6.71; N, 5.67%

Example 34 Preparation of cis-7-(1S-(3,5-dicarboxyphenylaminocarbonyl) -2-phenylethylaminocarbonyl) -8-(3,4-dichlorophenyl-methylaminocarbonyl) -2,3,5,6-dibenzobicyclo[2.2.2]octane Diastereoisomer 1

The compound was prepared essentially as in example 1 except that 3,4-dichlorobenzylamine was used in step b instead of 1-adamantanemethylamine.

The compound was further characterised and tested as the di-N-methyl-D-glucamine salt found: C, 56.58; H, 6.27; N, 5.90. C₅₆H₆₇Cl₂N₅O₁₇. 1.9 H₂O requires C, 56.65; H, 6.01; N, 5.89%

Example 35 Preparation of cis-7-(1S-(3,5-dicarboxyphenylaminocarbonyl)-2-phenylethylaminocarbonyl) -8-(3,4-dichlorophenyl-methylaminocarbonyl) -2,3,5,6-dibenzobicyclo[2.2.2]octane Diastereoisomer 2

The compound was prepared essentially as in example 34 except that the more polar material from the chromatography described in step f was used in the final hydrogenation.

The compound was further characterised and tested as the di-N-methyl-D-glucamine salt found: C, 56.98; H, 6.04; N, 6.05. C₅₆H₆₇Cl₂N₅O₁₇. 1.4 H₂O requires C, 57.08; H, 5.97; N, 5.94%

Example 36 Preparation of cis-7-(1S-(3,5-dicarboxyphenylaminocarbonyl) -3-phenylpropylaminocarbonyl) -8-(1-adamantanemethyl-aminocarbonyl) -2,3,5, 6-dibenzobicyclo[2.2.2]octane (mixture of diastereomers)

The compound was prepared essentially as in example 1 except that 2S- (tert-butyloxycarbonylamino)-4-phenylbutanoic acid was used in step e instead of BOC-L-phenylalanine and no attempt was made to separate the diastereomers in step f.

The compound was further characterised and tested as the di-N-methyl-D-glucamine salt found: C, 59.76; H, 7.03; N, 5.79. C₆₁H₈₁N₅O₁₇. 3.6 H₂O requires C, 60.01; H, 7.28; N, 5.74%

Example 37 Preparation of cis-7-(1R-(3,5-dicarboxyphenylaminocarbonyl) -3-phenylpropylaminocarbonyl)-8-(1-adamintanemethylaminocarbonyl)-2,3,5,6-dibenzobicyclo[2.2.2] octane (mixture of diastereomers)

The compound was prepared essentially as in example 1 except that 2R-(tert-butyloxycarbonylamino)-4-phenylbutanoic acid was used in step e instead of BOC-L-phenylalanine and no attempt was made to separate the diastereomers in step f.

The compound was further characterised and tested as the di-N-methyl-D-glucamine salt found: C, 59.65; H, 7.01; N, 5.51. C₆₁H₈₁N₅O₁₇. 3.7 H₂O requires C, 59.93; H, 7.28; N, 5.73%

Example 38 Preparation of cis-7-(1S-(3,5-dicarboxyphenylaminocarbonyl) -2-phenylethyl-N (methyl)-aminocarbonyl) -8-(1-adamantanemethylaminocarbonyl) -2,3,5, 6-dibenzobicyclo[2.2.2]octane (mixture of diastereomers)

The compound was prepared essentially as in example 1 except that N-methyl-BOC-L-phenylalanine was used in step e instead of BOC-L-phenylalanine and no attempt was made to separate the diastereomers in step f.

The compound was further characterised and tested as the di-N-methyl-D-glucamine salt found: C, 58.78; H, 7.09; N, 5.70. C₆₁H₈₁N₅O₁₇. 4.7 H₂O requires C, 59.04; H, 7.34; N, 5.64%

Example 39 Preparation of cis-7-(1S-(3,5-dicarboxyphenylaminocarbonyl) -3-phenylmethylaminocarbonyl)-8-(1-adamantanemethylaminocarbonyl) -2,3,5,6-dibenzobicyclo[2.2.2]octane (mixture of diastereomers)

The compound was prepared essentially as in example 1 except that BOC-L-phenylglycine was used in step e instead of BOC-L-phenylalanine and no attempt was made to separate the diastereomers in step f.

The compound was further characterised and tested as the N-methyl-D-glucamine salt found: C, 64.20; H, 6.75; N, 5.80. C₅₂H₆₀N₄O₁₂. 2.2 H₂O requires C, 64.21; H, 6.67; N, 5.76%

Example 40 Preparation of cis-7-(1S-(3-carboxyphenylaminocarbonyl)-2-phenylethylaminocarbonyl) -8-(1-adamantanemethylaminocarbonyl)-2,3,5,6-dibenzobicyclo[2.2.2]octane Diastereoisomer 1

The compound was prepared essentially as in example 1 steps f and g except that N-tert-butyloxycarbonyl-1S-(3-benzyloxycarbonyl-phenylaminocarbonyl)-2-phenylethylamine was used instead of N-tert-butyloxycarbonyl-1S-(3,5-dibenzyloxycarbonyl-phenyl aminocarbonyl)-2-phenylethylamine in step f. As before, the less polar compound after chromatography in step f was taken through to the compound of this example.

The compound was further characterised and tested as the N-methyl-D-glucamine salt found: C, 69.70; H, 7.00; N, 5.96. C₅₂H₆₂N₄O₁₀. 0.7 H₂O requires C, 69.45; H, 7.10; N, 6.23%

Example 41 Preparation of cis-7-(1S-(3-carboxyphenylaminocarbonyl)-2-phenylethylaminocarbonyl)-8-(1-adamantanemethylaminocarbonyl)-2,3,5,6-dibenzobicyclo[2.2.2]octane Diastereoisomer 2

The compound was prepared essentially as in example 40 except that the more polar.

The compound was further characterised and tested as the N-methyl-D-glucamine salt found: C, 66.60; H, 7.21; N, 6.06. C₅₂H₆₂N₄O₁₀. 1.9 H₂O requires C, 66.57 ; H, 7.08; N, 5.97%

Example 42 Preparation of cis-7-(1S-(3-carboxy-5-methoxycarbonyl-phenylaminocarbonyl)-2-phenylethylaminocarbcnyl)-8-(1-adamantanemethylaminocarbonyl) -2,3,5, 6-dibenzobicyclo[2.2.2]octane (mixture of diastereomers)

The compound was prepared essentially as in example 1 except that 3-methoxycarbonyl-5-nitrobenzoic acid was used in step c instead of 5-nitro-isophthalic acid.

The compound was further characterised and tested as the N-methyl-D-glucamine salt found: C, 65.81; H, 6.93; N, 5.79. C₅₄H₆₄N₄O₁₂. 1.5 H₂O requires C, 65.70; H, 6.83; N, 5.67%

Example 43 Preparation of cis-7-(1S-(3,4-dicarboxyphenylaminocarbonyl)-2-phenylethylaminocarbonyl)-8-(1-adamantanemethylaminocarbonyl)-2,3,5,6-dibenzobicyclo [2.2.2]octane (mixture of diastereomers)

The compound was prepared essentially as in example 1 except that 4-nitrophthalic acid was used in step c instead of 5-nitroisophthalic acid.

The compound was further characterised and tested as the di-N-methyl-D-glucamine salt found: C, 60.31; H, 7.22; N, 5.76. C₆₀H₇₉N₅O₁₇. 1.5 H₂O requires C, 60.29; H, 7.16; N, 5.86%

Example 44 Preparation of cis-7-(1S-(3,5-ditetrazolylphenylaminocarbonyl) -2-phenylethylaminocarbonyl) -8-(1-adamantanemethylaminocarbonyl)-2,3,5,6-dibenzobicyclo[2.2.2] octane (mixture of diastereomers)

The compound was prepared essentially as in example 1 except that the bis pivaloyloxymethyl (POM) derivative of N-tert-butyloxy-carbonyl-1S- (3,5-ditetrazolylphenylaminocarbonyl)-2-phenylethylamine was used instead of N-tert-butyloxycarbonyl-1S-(3,5-dibenzyloxycarbonylphenylaminocarbonyl) -2-phenylethylamine in step f and the deprotection in step g was performed with methanolic ammonia solution.

Example 45 (comparative) Preparation of cis-7-(1S-(2-carboxyphenylaminocarbonyl)-2-phenylethylaminocarbonyl)-8-(1-adamantane-methylaminocarbonyl) -2,3,5,6-dibenzobicyclo[2.2.2]octane (mixture of diastereomers)

The compound was prepared essentially as in example 1 steps f and g except that N-tert-butyloxycarbonyl-1S-(2-benzyloxycarbonylphenylaminocarbonyl)-2-phenylethylamine was used instead of N-tert-butyloxycarbonyl-1S- (3,5-dibenzyloxycarbonylphenylaminocarbonyl)-2-phenylethylamine in step f. No attempt was made to separate diastereoisomers.

The compound was further characterised and tested as the N-methyl-D-glucamine salt found: C, 63.51; H, 7.00; N, 5.52, C₅₂H₆₂N₄O₁₀. 4.2 H₂O requires C, 63.77; H, 7.25; N, 5.72%.

Example 46 (comparative) Preparation of cis-7-(1S-(4-carboxy-phenylaminocarbonyl) -2-phenylethylaminocarbonyl)-8-(1-adamantane-methylaminocarbonyl) -2,3,5,6-dibenzobicyclo[2.2.2]octane Diastereomer 1

The compound was prepared essentially as in example 1 steps f and g except that N-tert-butyloxycarbonyl-1S-(2-benzyloxycarbonyl-phenylaminocarbonyl)-2-phenylethylamine was used instead of N-tert-butyloxycarbonyl-1S- (3,5-dibenzyloxycarbonylphenylaminocarbonyl)-2-phenylethylamine in step f. The less polar material described from the chromatography in step f was taken through to the title compound of this example.

The compound was further characterised and tested as the N-methyl-D-glucamine salt found: C, 66.60; H, 7.21; N, 6.06. C₅₂H₆₂N₄O₁₀. 1.9 H₂O requires C, 66.57; H, 7.08; N, 5.97%.

Example 47 (comparative) Preparation of cis-7-(1S-(4-carboxyphenylaminocarbonyl)-2-phenylaminocarbonyl-8-(1-adamantanemethylaminocarbonyl) -2,3,5,6-dibenzobicyclo[2.2.2]octane Diastereomer 2

The more polar diastereomer described in example 46 was taken through as the title compound of this example.

The compound was further characterised and tested as the N-methyl-D-glucamine salt found: C, 62.98; H, 7.26; N, 6.02. C₅₂H₆₂N₄O₁₀ 4.6 H₂O requires C, 63.32; H, 7.28; N, 5.68%.

Example 48 (comparative) Preparation of cis-7-(1S-(3,5-dimethoxy-carbonylphenylaminocarbonyl)-2-phenylethylamino-carbonyl-8-(1-adamantanemethylaminocarbonyl)-2,3,5,6-dibenzobicyclo[2.2.2] octane Diastereomer 1

The compound of example 1 was treated with an excess of diazomethane to leave the title compound after quenching and evaporation found: C, 74.00; H, 6.40; N, 5.32. C₄₈H₄₉N₃O₇ requires C, 73.92; H, 6.33; N, 5.39%

Example 49 Preparation of cis-7-(1S-(3,5-dicarboxyphenylaminocarbonyl)-2-phenylethylaminocarbonyl)-8-(1-(1-adamantane)-1-methyl-ethylaminocarbonyl) -2,3,5,6-dibenzobicyclo[2.2.2]octane (mixture of diastereomers)

The compound was prepared essentially as in example 1 except that 1-(1-adamantane)-1-methylethylamine was used in step b instead of 1-adamantanemethylamine and no attempt was made to separate the diastereomers described in step f. m.p 195-8°.

The compound was further characterised and tested as the di N-methyl-D-glucamine salt found: C, 58.96; H, 7.33; N, 5.59. C₆₂H₈₃N₅O₁₇. 5 H₂O requires C, 659.08; H, 7.43; N, 5.56%

Example 50 Preparation of cis-7-(1S-(3,5-dicarboxyphenylaminocarbonyl) -2-phenylethylaminocarbonyl)-8-(3-indolylethylaminocarbonyl)-2,3,5,6-dibenzobicyclo[2.2.2] octane Diastereoisomer 1

The compound was prepared essentially as in example 1 except that 3-indolylethylamine was used in step b instead of 1-adamantanemethylamine.

The compound was further characterised and tested as the di N-methyl-D-glucamine salt found: C, 58.37; H, 6.44; N, 7.04. C₅₉H₇₂N₆O₁₇. 4 H₂O requires C, 58.60; H, 6.67; N, 5.95%

Example 51 Preparation of cis-7-(1S-(3,5-dicarboxyphenylaminocarbonyl) -2-phenylethylaminocarbonyl)-8-(3-indolylethylaminocarbonyl) -2,3,5,6-dibenzobicyclo [2.2.2]octane Diastereoisomer 2

The compound was prepared essentially as in example 50 except that the more polar material from the chromatography described in step f was used in the final hydrogenation.

The compound was further characterised and tested as the di N-methyl-D-glucamine salt found: C, 59.83; H, 6.20; N, 6.76. C₅₉H₇₂N₆O₁₇. 2.4 H₂O requires C, 60.03; H, 6.56; N, 7.12%

Example 52 Preparation of cis-7-(1S-(3,5-dicarboxyphenylaminocarbonyl) -2-phenylethylaminocarbonyl)-8-(2-thiophenylmethylaminocarbonyl)-2,3,5,6-dibenzobicyclo[2.2.2] octane (mixture of diastereomers)

The compound was prepared essentially as in example 1 except that 2-thiophenylmethylethylamine was used in step b instead of 1-adamantanemethylamine and no attempt was made to separate the diastereomers described in step f.

The compound was further characterised and tested as the di N-methyl-D-glucamine salt found: C, 52.68; H, 6.43; N, 5.67. C₅₄H₆₇N₆O₁₇S. 7.4 H₂O requires C, 52.99; H, 6.74; N, 5.72%

Example 53 Preparation of cis-7-(1S-(3,5-dicarboxyphenylaminocarbonyl) -2-phenylethylaminocarbonyl) -8-(2-phenylethylaminocarbonyl) -2,3,5,6-dibenzobicyclo[2.2.2]octane (mixture of diastereomers)

The compound was prepared essentially as in example 1 except that 2-phenylethylamine was used in step b instead of 1-adamantanemethylamine and no attempt was made to separate the diastereomers described in step f.

The compound was further characterised and tested as the di N-methyl-D-glucamine salt found: C, 59.15; H, 6.83; N, 5.81. C₅₇H₇₁N₅O₁₇. 3.5 H₂O requires C, 58.95; H, 6.77; N, 6.03%

Example 54 Preparation of cls-7-(1S-(3,5-dicarboxyphenylaminocarbonyl) -2-phenylethylaminocarbonyl) -8-(2-phenylpropylaminocarbonyl) -2,3,5,6-dibenzobicyclo[2.2.2]octane (mixture of diastereomers)

The compound was prepared essentially as in example 1 except that 3-phenylpropylamine was used in step b instead of 1-adamantanemethylamine and no attempt was made to separate the diastereomers described in step f.

The compound was further characterised and tested as the di N-methyl-D-glucamine salt found: C, 59.39; H, 6.79; N, 5.90. C₅₈H₇₃N₅O₁₇. 3.8 H₂O requires C, 59.38; H, 6.85; N, 5.96%

Example 55 Preparation of cis-7-(1S-(3-carboxyphenylaminocarbonyl)-2- (2-thiophenyl)ethylaminocarbonyl)-8-(1-adamantanemethylaminocarbonyl)-2,3,5,6-dibenzobicyclo[2.2.2]octane Diastereomer 1

The compound was prepared essentially as in example 1 except that step e was carried out using BOC-L-3-(2-thiophenyl)alanine instead of BOC-L-phenylalanine and 3-benzyloxycarbonylaniline instead of 3,5-dibenzyloxycarbonylaniline. The product of this reaction was used in step f instead of N-tert-butlyloxycarbonyl-1S-(3,5-dibenzyloxycarbonylphenylaminocarbonyl)-2-phenylethylamine.

The compound was further characterised and tested as the N-methyl-D-glucamine salt found: C, 66.21; H, 6.62; N, 5.87. C₅₀H₆₀N₄O₁₀S requires C, 66.06; H, 6.65; N, 6.16%

Example 56 Preparation of cis-7-(1S-13-carboxyphenylaminocarbonyl)-2- (2-thiophenyl)ethylaminocarbonyl) -8-(1-adamantanemethylaminocarbonyl)-2,3,5,6-dibenzobicyclo[2.2.2]octane Diastereomer 2 The compound was prepared essentially as in example 55 except that the more polar material from the chromatography described in step f was used in the final hydrogenation.

The compound was further characterised and tested as the N-methyl-D-glucamine salt found: C, 66.32; H, 6.81; N, 5.88. C₅₀H₆₀N₄O₁₀S requires C, 66.06; H, 6.65; N, 6.16%

Example 57 Preparation of cis-7-(1S-(3-carboxyphenylaminocarbonyl)-2- (2-thiophenyl)ethylaminocarbonyl)-8-(1-naphthalenemethylaminocarbonyl) -2,3,5,6-dibenzobicyclo[2.2.2]octane Diastereomer 1

The compound was prepared essentially as in example 55 except that 1 -naphthalenemethylamine was used in step b instead of 1-adamantanemethylamine.

The compound was further characterised and tested as the N-methyl-D-glucamine salt found: C, 58.71; H, 6.21; N, 5.85. C₅₀H₅₂N₄O₁₀.S. 6.4 H₂O requires C, 59.06; H, 6.43; N, 5.51%

Example 58 Preparation of cis-7-(1S-(3-carboxyphenylaminocarbonyl )-2- (2-thiophenyl)ethylaminocarbonyl)-8-(1-naphthalenemethylaminocarbonyl)-2,3,5,6- dibenzobicyclo[2.2.2]octane Diastereomer 2

The compound was prepared essentially as in example 57 except that the more polar material from the chromatography described in step f was used in the final hydrogenation.

The compound was tested as the N-methyl-D-glucamine salt.

Example 59 Preparation of cis-7-(1S-(3-carboxyphenylaminocarbonyl)-2-phenylethylaminocarbonyl)-8-(1-naphthalenemethylaminocarbonyl)-2,3,5,6-dibenzobicyclo[2.2.2]octane Diastereomer 1

The compound was prepared essentially as in example 57 except that BOC-L-phenylalanine was used in step e instead of BOC-L-3-(2-thiophenyl)alanine.

The compound was further characterised and tested as the N-methyl-D-glucamine salt found: C, 58.71; H, 6.21; N, 5.85. C₅₀H₅₂N₄O₁₀S. 6.4 H₂O requires C, 59.06; H, 6.43; N, 5.51%

Example 60 Preparation of cis-7-(1S-(3-carboxyphenylaminocarbonyl)-2-phenylethylaminocarbonyl)-8-(1-naphthalenemethylaminocarbonyl)-2,3,5,6-dibenzobicyclo [2.2.2]octane Diastereomer 2

The compound was prepared essentially as in example 59 except that the more polar material from the chromatography described in step f was used in the final hydrogenation.

The compound was tested as the N-methyl-D-glucamine salt.

Example 61 Preparation of cis-7-(1S-(3,5-dicarboxyphenylmethylaminocarbonyl) -2-phenylethylaminocarbonyl) -8- (1-adamantanemethylaminocarbonyl)-2,3,5,6-dibenzobicyclo [2.2.2] octane Diastereoisomer 1

The compound was prepared essentially as in example 1 except that 3,5-dibenzyloxycarbonylbenzylamine was used in step e instead of 3,5-dibenzyloxycarbonylaniline.

The compound was further characterised and tested as the N-methyl-D-glucamine salt found: C, 64.72; H, 6.96; N, 5.88. C₅₄H₆₄N₄O₁₂. 2 H₂O requires C, 64.99; H, 6.88; N, 5.61%

Example 62 Preparation of cis-7-(1S-(3,5-dicarboxyphenylmethylaminocarbonyl)-2-phenylethylaminocarbonyl)-8-(1-adamantanemethyl-aminocarbonyl)-2,3,5, 6-dibenzobicyclo[2.2 .2]octane Diastereoisomer 2

The compound was prepared essentially as in example 61 except that the more polar material from the chromatography described in step f was used in the final hydrogenation.

The compound was further characterised and tested as the N-methyl-D-glucamine salt found: C, 64.99; H, 6.95; N, 5.65. C₅₄H₆₄N₄O₁₂. 2 H₂O requires C, 64.99; H, 6.88; N, 5.61%

Example 63 Preparation of cis-7-(1S-(3,5-dicarboxyphenylaminocarbonyl) -2-phenylethylaminocarbonyl)-8-(2-naphthalenemethylaminocarbonyl) -2,3,5,6-dibenzobicyclo[2.2.2]octane Diastereoisomer 1

The compound was prepared essentially as in example 1 except that 2-naphthalenemethylamine was used in step b instead of 1-adamantanemethylamine, found: C, 73.99; H, 5.26; N, 5.41. C₄₆H₃₇N₃O₇.requires C, 74.28; H, 5.01; N, 5.65%

The compound was tested as the di N-methyl-D-glucamine salt.

Example 64 Preparation of cis-7-(1S-(3,5-dicarboxyphenylaminocarbonyl) -2-phenylethylaminocarbcnyl)-8- (2-naphthalenemethylaminocarbonyl)-2,3,5,6-dibenzobicyclo [2.2.2]octane Diastereoisomer 2

The compound was prepared essentially as in example 63 except that the more polar material from the chromatography described in step f was used in the final hydrogenation found: C, 72.26; H, 5.18; N, 5.42. C₄₆H₃₇N₃O_{7.} 1.1 H₂O requires C, 72.30; H, 5.18; N, 5.50%

The compound was tested as the di N-methyl-D-glucamine salt.

The compounds of the examples gave the following ¹H NMR spectra:
Ex.1a (d⁶-DMSO) δ 7.5 (2H, m), 7.3 (2H, m), 7.2 (4H, m), 4.8 (2H, s), 3.6 (2H,s)
Ex.1b (d⁷-DMF) δ 7.7 (1H, t), 7.4 (3H, m), 7.2 (3H,m), 7.1 (2H, m), 4.7 (1H, d), 4.6 (1H, d), 3.5 (1H, dd), 3.0 (1H, dd), 2.9 (1H, dd), 2.7 (1H, dd), 2.0 (3H, s), 1.7 (6H, m), 1.5 (6H, s)
Ex.1c (CDCl₃) δ 9.C (3H, d), 7.5 (10H, m), 5.5 (4H, s)
Ex.1d (CDCl₃) δ 8.1 (1H, d), 7.5 (12H, m), 5.4 (4H, s), 3.8 (2H, bs)
Ex.1e (d⁶-DMSO) δ 10.5 (1H, s), 8.5 (2H, s), 8.2 (1H, s), 7.3 (15H, m), 5.4 (4H, s), 4.3 (1H, m), 2.9 (2H, m), 1.3 (9H,s
Ex.1g (d⁶-DMSO) δ 9.8 (1H, s), 8.4 (2H, s), 8.1 (1H, s), 8.0 (1H, d), 7.1 (14H, m), 4.5 (2H, s), 4.2 (1H, s), 3.1 (2H, m), 2.8 (2H, m), 2.6 (1H, m), 2.2 (1H, m), 1.7 (3H, s), 1.5 (6H, m), 1.1 (6H, s)
Ex.2 (d⁶-DMSO) δ 9.9 (1H, s), 8.3(2H, s), 8.1 (1H, s), 7.1 (15H, m), 4.4 (3H, m), 3.1 (2H, m), 2.8 (2H, m),2.6 (1H, m), 2.2 (1H, m), 1.8 (3H, s), 1.5 (6H, m), 1.2 (6H, s)
Ex.3 (d⁶-DMSO) δ 9.8 (1H, s), 8.4 (2H, s), 8.1 (1H, s), 8.0 (1H, d), 7.1 (14H, m), 4.5 (2H, s), 4.2 (1H, s), 3.1 (2H, m), 2.8 (2H, m), 2.6 (1H, m), 2.2 (1H, m), 1.7 (3H, s), 1.5 (6H, m), 1.1 (6H, s)
Ex.4 (d⁶-DMSO) δ 9.9 (1H, s), 8.3 (2H, s), 8.1 (1H, s), 7.1 (15H, m), 4.4 (3H, m), 3.1 (2H, m), 2.8 (2H, m), 2.6 (1H, m), 2.2 (1H, m), 1.8 (3H, s), 1.5 (6H, m), 1.2 (6H, s)
Ex.5 (d⁶-DMSO) δ 10.1 and 9.8 (1H, 2 x s), 8.4 (2H, d), 8.1 (1H, m), 7.7 and 7.4 (1H, 2 xm), 7.1 (9H, m), 4.5 (2H, m), 4.2 (1H, m), 3.2 (2H, m), 2.9 (1H, m), 2.6 (1H, m), 2.2 (1H, m), 1.8-0.8 (28H, m)
Ex.6 (d⁶-DMSO) δ 9.8 (1H, s), 8.4 (2H, s), 8.1 (2H, s), 7.2 (12H, m), 4.4 (3H, m), 3.0 (4H, m), 2.6 (1H, m), 2.2 (1H, m), 1.7 (3H, s), 1.5 (6H, m), 1.1 (6H, s)
Ex.7 (d⁶-DMSO) δ 9.9 (1H, s), 8.3 (2H, s), 8.1 (1H, s), 7.1 (13H, m), 4.5 (3H, m), 3.1 (2H, m), 2.8 (2H, m), 2.6 (1H, m), 2.2 (1H, m), 1.8 (3H, s), 1.6 (6H, m), 1.2 (6H, m)
Ex.8 (d⁶-DMSO) δ 9.8 (1H, s), 8.4 (2H, s), 8.1 (1H, s), 8.0 (1H, d), 7.2 (13H, m), 4.5 (2H, m), 4.2 (1H, s), 3.1 (2H, m), 2.8 (2H, m), 2.6 (1H, m), 2.2 (1H, m), 1.7 (3H, s), 1.5 (6H, m), 1.1 (6H, s)
Ex.9 (d⁶-DMSO) δ 9.9 (1H, s), 8.3 (2H, s), 8.1 (1H, s), 7.1 (14H, m), 4.5 (3H, m), 3.1 (1H, m), 2.8 (3H, m), 2.4 (2H, m), 1.8 (3H, s), 1.5 (6H, m), 1.2 (6H, m)
Ex.10 (d⁶-DMSO) δ 9.8 (1H, s), 8.4 (2H, s), 8.1 (1H, s), 8.0 (1H, d), 7.2 (13H, m), 4.5 (2H, m), 4.2 (1H, m), 3.1 (2H, m), 3.1 (2H, m), 2.6 (1H, m), 2.2 (1H, m), 1.7 (3H, s), 1.5 (6H, m), 1.1 (6H, s)
Ex.11 (d⁶-DMSO) δ 9.9 (1H, s), 8.3 (2H, s), 8.1 (1H, s), 7.1 (14H, m), 4.5 (3H, m), 3.1 (1H, m), 2.9 (3H, m), 2.4 (2H, m), 1.8 (3H, s), 1.5 (6H, m), 1.2 (6H, m)
Ex.12 (d⁶-DMSO) δ 9.8 (1H, s), 8.4 (2H, s), 8.1 (1H, s), 7.9 (1H, d), 7.2 (13H, m), 4.4 (2H, m), 4.2 (1H, m), 3.7 (3H, s), 3.0 (4H, m), 2.6 (1H, m), 2.2 (1H, m), 1.7 (3H, s), 1.5 (6H, m), 1.1 (6H, s)
Ex.13 (d⁶-DMSO) δ 9.9 (1H, s), 8.3 (2H, s), 8.1 (1H, s), 7.1 (14H, m), 4.4 (3H, m), 3.7 (3H, s), 3.1 (2H, m), 2.9(2H, m), 2.4 (2H, m), 1.8 (3H, s), 1.6 (6H, m), 1.2 (6H, m)
Ex.14 (d⁶-DMSO) δ 13.2 (2H, br s), 9.9 (1H, s), 8.4 (2H, s), 8.1 (1H, s), 8.0 (1H, d), 7.9-6.8 (16H, m), 4.5 (2H, m), 4.0 (1H, s), 3.1 (2H, m), 2.8 (2H, m), 2.6 (1H, m), 2.2 (1H, m), 1.7 (3H, s), 1.5 (6H, m), 1.1 (6H, s)
Ex.15 (d⁶-DMSO) δ 13.1 (2H, br s), 9.9 (1H, s), 8.3 (2H, s), 8.1 (1H, s), 7.9-6.8 (17H, m), 4.5 (3H, m), 3.1 (1H, m), 2.8 (3H, m), 2.4 (2H, m), 1.8 (3H, s), 1.5 (6H, m), 1.2 (6H, m)
Ex.16 (d⁶-DMSO) δ 13.2 (2H, br s), 9.8 (1H, s), 8.4 (2H, s), 8.1 (1H, s), 8.0 (1H, d), 7.2 (13H, m), 4.5 (2H, m), 4.2 (1H, s), 3.1 (2H, m), 2.8 (2H, m), 2.6 (1H, m), 2.2 (1H, m), 1.7 (3H, s), 1.5 (6H, m), 1.1 (6H, s)
Ex.17 (d⁶-DMSO) δ 13.2 (2H, br s), 9.9 (1H, s), 8.3 (2H, s), 8.1 (1H, s), 7.1 (14H, m), 4.5 (3H, m), 3.1 (1H, m), 2.8 (3H, m), 2.4 (2H, m), 1.8 (3H, s), 1.5 (6H, m), 1.2 (6H, m)
Ex.18 (d⁶-DMSO) δ 13.2 (2H, br s), 9.8 (1H, s), 8.4 (2H, s), 8.1 (1H, s), 8.0 (1H, d), 7.2 (13H, m), 4.5 (2H, m), 4.2 (1H, s), 3.1 (2H, m), 2.8 (2H, m), 2.6 (1H, m), 2.2 (1H, m), 1.7 (3H, s), 1.5 (6H, m), 1.1 (6H, s)
Ex.19 (d⁶-DMSO) δ 13.2 (2H, br s), 9.9 (1H, s), 8.3 (2H, s), 8.1 (1H, s), 7.1 (14H, m), 4.5 (3H, m), 3.1 (1H, m), 2.8 (3H, m), 2.4 (2H, m), 1.8 (3H, s), 1.5 (6H, m), 1.2 (6H, m)
Ex.20 (d⁶-DMSO) δ 13.2 (2H, br s), 11.1 and 9.8 (1H, 2 x s), 8.2 (4H, m), 7.2 (13H, m), 4.5-4.2 (3H, m), 3.2-2.4 (6H, m), 1.7-1.1 (15H, m)
Ex.21 (d⁶-DMSO) δ 9.9 (1H, s), 8.3 (2H, s), 8.1 (1H, s), 7.1 (13H, m), 4.5 (3H, m), 3.1 (2H, m), 2.8 (2H, m), 2.6 (1H, m), 2.2 (1H, m), 1.8 (3H, s), 1.6 (6H, m), 1.2 (6H, m)
Ex.22 (CDCl₃) δ 8.6 (1H, s), 7.6 (2H, d), 7.2 (16H, m), 5.8 (1H, m), 5.6 (1H, m), 4.8 (1H, m), 4.5 (1H, d), 4.2 (1H, d), 3.2 (3H, m), 3.0 (1H, dd), 2.6 (1H, m), 2.4 (1H, m), 1.9 (3H, s), 1.6 (6H, m), 1.2 (6H, s)
Ex.23 (CDCl₃) δ 8.8 (1H, s), 7.3 (18H, m), 6.1 (1H, m), 6.0 (1H, m), 5.0 (1H, m), 4.4 (1H, d), 4.1 (1H, d), 3.7 (1H, dd), 3.2 (1H, m), 3.1-2.8 (3H, m), 2.4 (1H, m), 2.0 (3H, s), 1.7 (6H, m), 1.3 (6H, s)
Ex.24 (d⁶-DMSO) δ 9.8 (1H, s),9.2 (1H, br s), 8.4 (2H, s), 8.1 (1H, s), 8.0 (1H, d), 7.2 (11H, m), 6.6 (2H, d), 4.5 (1H, s), 4.4 (1H, m), 4.2 (1H, s), 3.1 (2H, m), 2.8 (2H, m), 2.6 (1H, m), 2.2 (1H, m), 1.7 (3H, s), 1.5 (6H, m), 1.1 (6H, s)
Ex.25 (d⁶-DMSO) δ 9.8 (1H., s), 9.2 (1H, s), 8.3 (2H, s), 8.1 (1H, s), 7.1 (12H, m), 6.6 (2H, d), 4.4 (3H, m), 3.1 (1H, m), 2.8 (3H, m), 2.4 (2H, m), 1.8 (3H, s), 1.5 (6H, m), 1.2 (6H, m)
Ex.26 (d⁶-DMSO) δ 9.8 (1H, s),9.2 (1H, br s), 8.4 (2H, s), 8.1 (1H, s), 8.0 (1H, d), 7.2 (11H, m), 6.6 (2H, d), 4.5 (1H, s), 4.4 (1H, m), 4.2 (1H, s), 3.1 (2H, m), 2.8 (2H, m), 2.6 (1H, m), 2.2 (1H, m), 1.7 (3H, s), 1.5 (6H, m), 1.1 (6H, s)
Ex.27 (d⁶-DMSO) δ 9.8 (1H, s),9.2 (1H, br s), 8.4 (2H, s), 8.1 (1H, s), 8.0 (1H, d), 7.2 (11H, m), 6.6 (2H, d), 4.5 (1H, s), 4.4 (1H, m), 4.2 (1H, s), 3.1 (2H, m), 2.8 (2H, m), 2.6 (1H, m), 2.2 (1H, m), 1.7 (3H, s), 1.5 (6H, m), 1.1 (6H, s)
Ex.28 (d⁶-DMSO) δ 9.8 (1H, s), 8.4 (2H, s), 8.1 (1H, s), 8.0 (1H, d), 7.1 (14H, m), 4.5 (2H, m), 4.2 (1H, s), 3.1 (3H, m), 2.7 (3H, m), 1.4-0.7 (13H, m)
Ex.29 (d⁶-DMSO) δ 9.9 (1H, s), 8.3 (2H, s), 8.1 (1H, s), 7.1 (15H, m), 4.5 (3H, m), 3.1 (4H, m), 2.6 (2H, m), 1.4-0.7 (13H, m)
Ex.30 (d⁶-DMSO) δ 9.8 (1H, s), 8.4 (2H, s), 8.1 (1H, s), 8.0 (1H, d), 7.1 (14H, m), 4.5 (2H, m), 4.2 (1H, s), 3.1 (3H, m), 2.7 (3H, m), 1.4-0.7 (11H, m)
Ex.31 (d⁶-DMSO) δ 9.9 (1H, s), 8.3 (2H, s), 8.1 (1H, s), 7.1 (15H, m), 4.5 (3H, m), 3.0 (4H, m), 2.6 (2H, m), 1.6-0.6 (11H, m)
Ex.32 (d⁶-DMSO) δ 13.1 (2H, br s), 9.8 (1H, s), 8.4 (2H, s), 8.3-6.8 (23H, m), 4.7-4.2 (3H, m), 3.2 (4H, m), 2.7 (2H, m)
Ex.33 (d⁶-DMSO) δ 13.2 (2H, br s), 10.0 (1H, s), 8.3 (2H, s), 8.2-6.8 (23H, m), 4.5 (3H, m), 3.2 (3H, m), 2.7 (3H, m)
Ex.34 (d⁶-DMSO) δ 9.6 (1H, s), 8.4-6.8 (21H, m), 4.7-4.1 (3H, m), 3.2 (4H, m), 2.8 (2H, m)
Ex.35 (d⁶-DMSO) δ 9.8 (1H, s), 8.3-6.7 (21H, m), 4.5-4.1 (3H, m), 3.2 (4H, m), 2.9 (2H, m)
Ex.36 (d⁶-DMSO) δ 13.1 (2H, br s), 10.1-9.7 (1H, 3 x s), 8.4-7.0 (18H, m), 4.5-4.1 (3H, m), 3.1 (2H, m), 2.8 (2H, m), 2.6 (2H, m), 2.0 (2H, m), 1.7 (3H, s), 1.5 (6H, m), 1.1 (6H, m)
Ex.37 (d⁶-DMSO) δ 13.1 (2H, br s), 10.1-9.7 (1H, 3 x s), 8.4-7.0 (18H, m), 4.5-4.1 (3H, m), 3.1 (2H, m), 2.8 (2H, m), 2.6 (2H, m), 2.0 (2H, m), 1.7 (3H, s), 1.5 (6H, m), 1.1 (6H, m)
Ex.38 (d⁶-DMSO) δ 13.2 (2H, br s), 11.0 and 10.9 (1H, 2 x s), 9.9 and 9.7 (1H, 2 x s), 8.4-6.8 (15H, m), 6.5 and 6.3 (1H, 2 x t), 4.5-4.1 (3H, m), 3.2 (5H, m), 2.8 (2H, m), 2.6 (1H, m), 2.2 (1H, m), 1.7 (3H, m), 1.5 (6H, m), 1.1 (6H, m)
Ex.39 (d⁶-DMSO) δ 10.5 and 10.3 (1H, 2 x s), 8.4-8.0 (3H, m), 7.2 (13H, m), 5.3 (1H, d), 4.5 (2H, m), 3.1 (2H, m), 2.5 (2H, m), 1.8 (3H, m), 1.5 (6H, m), 1.2 (6H, m)
Ex.40 (d⁶-DMSO) δ 9.7 (1H, s), 8.2 (1H, s), 8.0 (1H, m), 7.8-6.9 (17H, m), 4.5 (1H, s), 4.4 (1H, s), 4.2 (1H, s), 3.5-3.2 (2H, m), 2.9 (2H, m), 2.6 (1H, m), 2.3 (1H, m), 1.7 (3H, s), 1.5 (6H, m), 1.4 (6H, s)
Ex.41 (d⁶-DMSO) δ 12.9 (1H, s), 9.7 (1H, s), 8.2 (1H, s), 7.6 (2H, t), 7.4-7.2 (11H, m), 7.1 (2H, m), 7.0 (2H, q), 6.8 (1H, t), 4.5 (3H, m), 3.2 (1H, dd), 3.0 (2H, m), 2.9 (1H, dd), 2.6 (1H, dd), 2.4 (1H, dd), 1.8 (3H, s), 1.6 (6H, m), 1.3 (6H, s)
Ex.42 (d⁶-DMSO) δ 9.2-7.7 (5H, m), 7.3 (13H, m), 7.0 and 6.8 (1H, 2 x t), 5.0-4.2 (3H, m), 3.8 (3H, m), 3.4-2.5 (6H, m), 1.9 (3H, m), 1.5 (6H, m), 1.1 (6H, m)
Ex.43 (d⁶-DMSO) δ 13.0 (2H, br s), 9.8 (1H, d), 8.0 (1H, d), 7.9-6.8 (17H, m), 4.5-4.2 (3H, m), 3.1 (2H, m), 2.8 (2H, m), 2.6 (1H, m), 2.3 (1H, m), 1.8 (3H, m), 1.5 (6H, m), 1.1 (6H, m)
Ex.44 (d⁶-DMSO) δ 9.8 (1H, m), 8.4-8.0 (3H, m), 7.1 (14H, m), 4.5 (3H, m), 3.0 (4H, m), 2.6 (1H, m), 2.2 (1H, m), 1.8 (3H, m), 1.5 (6H, m), 1.1 (6H, s)
Ex.45 (d⁶-DMSO) δ 13.7 (1H, br s), 8.5 (2H, m), 8.2-7.9 (2H, m), 7.6-6.4 (16H, m), 4.5 (1H, s), 4.4 (1H, m), 4.2 (1H, s), 3.5-3.2 (2H, m), 2.9 (2H, m), 2.6 (1H, m), 2.3 (1H, m), 1.7 (3H, m), 1.5 (6H, m), 1.0 (6H, m)
Ex.46 (d⁶-DMSO) δ 12.9 (1H, br s), 9.7 (1H, s), 8.2 (1H, s), 7.6-7.0 (17H, m), 6.8 (1H, t), 4.5 (3H, m), 3.2 (1H, m), 3.0 (2H, m), 2.9 (1H, m), 2.4 (1H, m), 1.8 (3H, m), 1.5 (6H, m), 1.3 (6H, m)
Ex.47 (d⁶-DMSO) δ 9.8 (1H, s), 8.0 (1H, s), 7.9 (1H, d), 7.8-6.9 (17H, m), 4.5 (2H, m), 4.2 (1H, m), 3.4-3.1 (2H, m), 2.8 (1H, d), 2.7-2.3 (3H, m), 1.9 (3H, m), 1.5 (6H, m), 1.2 (6H, m)
Ex.48 (d⁶-DMSO) δ 9.8 (1H, s), 8.4 (2H, s), 8.1 (1H, s), 8.0 (1H, d), 7.1 (14H, m), 4.5 (2H, s), 4.2 (1H, s), 3.9 (6H, s), 3.1 (2H, m), 2.8 (2H, m), 2.6 (1H, m), 2.2 (1H, m), 1.7 (3H, s), 1.5 (6H, m), 1.1 (6H, s)
Ex.49 (CDCl₃) δ 9.2 and 9.1 (1H, 2 x s), 8.4-8.0 (4H, m), 7.1 (14H, m), 4.8 (1H, m), 4.5 (2H, m), 3.6-2.6 2.6 (4H, m), 1.7 (3H, m), 1.5 (6H, m), 1.1 (6H, m), 0.7 (6H, m)
Ex.50 (d⁶-DMSO) δ 10.5 (1H, s), 9.9 (1H, s), 8.5 (2H, s), 8.1 (1H, s), 7.9 (1H, d), 7.7 (1H, br t), 7.4-6.7 (18H, m), 4.6 (1H, m), 4.4 (1H, s), 4.1 (1H, s), 3.3 (2H, m), 3.0 (2H, m), 2.9-2.7 (2H, m), 2.6 (2H, m)
Ex.51 (d⁶-DMSO) δ 13.2 (2H, br s), 10.8 (1H, s), 10.0 (1H, s), 8.3 (2H, d), 8.1 (1H, d), 7.6 (1H, br t), 7.5-6.8 (19H, m), 4.5 (1H, m), 4.45 (1H, d), 4.37 (1H, d), 3.3-2.8 (6H, m), 2.7 (2H, m)
Ex.52 (d⁶-DMSO) δ 9.9 (1H, s), 8.5 (2H, s), 8.2 (1H, br s), 8.1 (1H, s), 7.4-6.7 (17H, m), 4.5 (2H, m), 4.2 (3H, m), 3.0 (4H, m)
Ex.53 (d⁶-DMSO) δ 13.2 (2H, br s), 10.0 (1H, br s), 8.5 (2H, s), 8.2 (1H, br s), 8.1 (1H, s), 7.7 (1H, br s), 7.5-6.8 (18H, m), 4.5 (3H, m), 3.0 (6H, m), 2.5 (2H, m)
Ex.54 (d⁶-DMSO) δ 13.2 (2H, br s), 10.0 (1H, 2xs), 8.3 (2H, s), 8.1 (1H, br s), 8.0 (1H, d), 7.5-6.8 (19H, m), 4.5 (3H, m), 3.0 (6H, m), 2.0 (2H, m), 1.5 (2H, t)
Ex.55 (d⁶-DMSO) δ 13.2 (1H, br s), 9.7 (1H, s), 8.2 (1H, s), 8.0 (1H, br s), 7.8 (1H, d), 7.6 (1H, d), 7.4-6.9 (13H, m), 4.5 (3H, m), 3.0 (4H, m), 2.4 (2H, m), 1.8 (3H, d), 1.5 (6H, m), 1.2 (6H, m)
Ex.56 (d⁶-DMSO) δ 13.0 (1H, br s), 9.7 (1H, s), 8.2 (1H, s), 7.6 (2H, m), 7.4-6.8 (14H, m), 4.5 (3H, m), 3.0 (4H, m), 2.4 (2H, m), 1.8 (3H, s), 1.5 (6H, q), 1.2 (6H, s)
Ex.57 (d⁶-DMSO) δ 13.0 (1H, br s), 9.7 (1H, s), 8.3 (1H, s), 8.2 (1H, br s), 8.0-6.9 (22H, m), 4.7-4.2 (5H, m), 3.0 (4H, m)
Ex.58 (d⁶-DMSO) δ 13.0 (1H, br s), 9.8 (1H, s), 8.2 (2H, br s), 8.0-6.9 (22H, m), 4.7-4.2 (5H, m), 3.0 (4H, m)
Ex.59 (d⁶-DMSO) δ 12.8 (1H, br s), 9.8 (1H, s), 8.3 (1H, br s), 8.2 (1H, s), 8.0-6.9 (24H, m), 4.7-4.2 (5H, m), 3.0 (4H, m)
Ex.60 (d⁶-DMSO) δ 12.8 (1H, br s), 9.8 (1H, s), 8.4 (1H, br s), 8.3 (1H, s), 8.0-6.9 (24H, m), 4.7-4.2 (5H, m), 3.0 (4H, m)
Ex.61 (d⁶-DMSO) δ 8.3 (2H, m), 8.0 (2H, s), 7.8 (1H, d), 7.4 (1H, t), 7.3-6.9 (13H, m), 4.5 (1H, s), 4.3 (3H, m), 4.1 (1H, s), 3.2-2.4 (6H, m), 1.8 (3H, m), 1.5 (6H, m), 1.2 (6H, m)
Ex.62 (d⁶-DMSO) δ 8.3 (2H, m), 8.0 (2H, s), 7.4-7.0 (12H, t), 6.9 (3H, m), 4.4 (2H, 2xs), 4.3 (1H, m), 4.2 (2H, m), 3.2-2.4 (6H, m), 1.8 (3H, m), 1.6 (6H, m), 1.2 (6H, m)
Ex.63 (d⁶-DMSO) δ 13.2 (2H, br s), 9.8 (1H, s), 8.4 (2H, s), 8.3 (1H, m), 8.1 (2H, m), 7.7 (3H, m), 7.5 (1H, s), 7.3 (9H, m), 7.2 (2H, m), 7,1 (2H, m), 6.9 (3H, s), 4.6 (1H, s), 4.55 (1H, m), 4.4 (1H, m), 4.3 (1H, s), 4.1 (1H, m), 3.2 (2H, m), 2.9 (1H, m), 2.8 (1H, m)
Ex.64 (d⁶-DMSO) δ 13.2 (2H, br s), 9.9 (1H, s), 8.4 (2H, s), 8.3 (1H, m), 8.1 (2H, m), 7.7 (3H, m), 7.5 (1H, s), 7.3 (9H, m), 7.2 (2H, m), 7.1 (2H, m), 6.9 (3H, s), 4.6 (1H, s), 4.55 (1H, m), 4.4 (1H, m), 4.3 (1H, s), 4.1 (1H, m), 3.2 (2H, m), 2.9 (1H, m), 2.8 (1H, m)

The compounds of the examples were tested for binding at the CCK_{B} receptor in mouse cortical membranes by means of a radioligand binding assay. The procedure was as follows:

The whole brains from male mice (CD1 22-25g; Charles River) were removed and placed in ice-cold buffer (pH7.2 @ 21 ± 3°C) of the following composition (mM); 10 HEPES, 130 NaCl, 4.7 KCl, 5 MgCl₂, 1 EDTA and containing 0.25g.l⁻¹ bacitracin. The cortex was dissected, weighed and homogenised in 40ml ice-cold buffer using a Teflon-in-glass homogeniser. The homogenate was centrifuged at 39,800g for 20 min at 4°C, the supernatant discarded and the pellet resuspended by homogenisation in fresh buffer. The homogenate was recentrifuged (39,800g; 20 min @ 4°C) and the final pellet was resuspended in HEPES buffer to give a tissue concentration of 2mg.ml⁻¹(original wet weight).

The membranes (400ml) were incubated for 150 min at 21 ± 3°C in a final volume of 0.5ml with HEPES buffer containing [¹²⁵I]-CCK8S (0.05ml; 200pM NEN 2200Ci.mmol⁻¹) and competing compound. Total and non-specific binding of [¹²⁵I]-CCK8S were defined using 0.05ml of buffer and 0.05ml of 10mM L-365,260, respectively. The assay was terminated by rapid filtration through pre-soaked Whatman GF/B filters using a Brandell Cell harvester. The filters were washed (3 x 3ml) with ice-cold 50mM Tris-HCl (pH7.4 @ 4°C) and bound radioactivity determined by counting (1 min.) in a gamma-counter.

The results obtained from the CCK_{B} assays are set out in Table 1.

**TABLE 1**

| Example | CCK_{B} pKᵢ | Example | CCK_{B} pKᵢ |
|---|---|---|---|
| 1 | 8.8 | 34 | 7.4 |
| 2 | 7.8 | 35 | 6.8 |
| 3 | 8.0 | 36 | 7.2 |
| 4 | 7.8 | 37 | 7.0 |
| 5 | 7.6 | 38 | 7.3 |
| 6 | 8.6 | 39 | 6.3 |
| 7 | 7.3 | 40 | 7.8 |
| 8 | 7.9 | 41 | 6.8 |
| 9 | 7.3 | 42 | 7.5 |
| 10 | 8.0 | 43 | 7.0 |
| 11 | 6.6 | 44 | 6.5 |
| 12 | 7.0 | 45* | 6.7 |
| 13 | 6.3 | 46* | 6.8 |
| 14 | 6.5 | 47* | 6.9 |
| 15 | 6.7 | 48* | 5.0 |
| 16 | 8.5 | 49 | 7.2 |
| 17 | 8.2 | 50 | 7.8 |
| 18 | 8.5 | 51 | 7.1 |
| 19 | 8.0 | 52 | 7.3 |
| 20 | 8.0 | 53 | 8.4 |
| 21 | 7.3 | 54 | 7.5 |
| 22* | 5.4 | 55 | 8.2 |
| 23* | 5.4 | 56 | 7.6 |
| 24 | 8.8 | 57 | 7.2 |
| 27 | 7.8 | 58 | 6.3 |
| 28 | 8.4 | 59 | 7.2 |
| 29 | 7.4 | 60 | 6.4 |
| 30 | 7.7 | 61 | 7.5 |
| 31 | 6.7 | 62 | 7.4 |
| 32 | 8.0 | 63 | 8.6 |
| 33 | 6.5 | 64 | 8.3 |

| | | | |
|---|---|---|---|
| * - comparative examples | | | |

The compounds of the examples were also tested for gastrin antagonist activity in an immature rat stomach assay. The procedure was as follows:

The oesophagus of immature rats (33-50 g, ca. 21 days old) was ligated at the level of the cardiac sphincter and the duodenal sphincter was cannulated. The stomach was excised and flushed with ca. 1 ml of unbuffered physiological saline solution. The fundus was punctured and cannulated. A further 4-5 ml of unbuffered solution was flushed through the stomach to ensure the preparation was not leaking. The stomach was lowered into a jacketed organ bath containing 40 ml of buffered solution containing 3x10⁻⁸M 5-methylfurmethide, maintained at 37° and gassed vigorously with 95% O₂/ 5% CO₂. The stomach was continuously perfused at a rate of 1 ml min⁻¹ with unbuffered solution gassed with 100% O₂ with the perfusate passing over an internally referenced pH-electrode fixed 12 cm above the stomach.

After 120 min of stabilisation the drugs were added directly to the serosal solution in the organ bath and after a further 60 min cumulative pentagastrin dose-response curves were started. Changes in acid secretion were monitored and the curves analysed according to Black et.al., Br. J. Pharmacol., 1985, 86, 581.

The results obtained from the gastrin assays are set out in Table 2.

**TABLE 2**

| Example | Gastrin pK_{B} | Example | Gastrin pK_{B} |
|---|---|---|---|
| 1 | 8.3 | 28 | 8.4 |
| 2 | 7.3 | 29 | 7.0 |
| 3 | 6.9 | 30 | 7.3 |
| 4 | 7.8 | 31 | 6.6 |
| 5 | 6.5 | 32 | 7.6 |
| 6 | 7.9 | 34 | 6.5 |
| 7 | 6.6 | 35 | 6.0 |
| 8 | 8.1 | 36 | 7.5 |
| 9 | 7.7 | 37 | 7.2 |
| 10 | 8.3 | 38 | 7.1 |
| 11 | 7.0 | 39 | 6.4 |
| 12 | 7.2 | 40 | 5.7 |
| 13 | 6.5 | 41 | 5.7 |
| 14 | 6.9 | 42 | 5.8 |
| 15 | 5.8 | 43 | 6.1 |
| 16 | 8.6 | 44 | 6.9 |
| 17 | 8.0 | 45* | - |
| 18 | 8.4 | 46* | 5.7 |
| 19 | 7.6 | 47* | - |
| 20 | 8.2 | 48* | - |
| 21 | 7.2 | 49 | 8.0 |
| 23* | 6.6 | 53 | 7.1 |
| 24 | 8.9 | 61 | 7.4 |
| 25 | 7.2 | 62 | 7.2 |
| 26 | 6.8 | 63 | 7.4 |
| 27 | 8.1 | 64 | 6.5 |

| | | | |
|---|---|---|---|
| * - comparative examples | | | |

The compounds of the examples were also tested in a CCKÅ binding assay as follows:

The pancreatata were removed from male guinea-pigs (200-300g; Dunkin Hartley) and placed in ice-cold HEPES buffer (pH 7.2 @ 21 ± 3° ). The pancreatata were homogenised in 40 ml ice-cold HEPES buffer using a polytron (Brinkmann, PT10, setting 10) 4 x 1 second.

The homogenate was centrifuged at 39,800g for 15 min at 4°. The supernatant was discarded and the pellet re-suspended using a Teflon-in-glass homogeniser in 20 volumes of fresh buffer and recentrifuged as above. The final pellet was re-suspended using a Teflon-in-glass homogeniser to a tissue concentration of 1 mg.ml⁻¹ (original wet weight), and filtered through 500 µm pore-size Nytex mesh.

The membranes (400µl; containing 0.375 µM PD134,308) are incubated for 150 minutes at 21 ± 3° in a final volume of 0.5ml with HEPES buffer containing [¹²⁵I]-CCK₈(S) (50µl; 200pM) and competing compound. Total and non-specific binding of [¹²⁵I]-CCK₈ (S) are defined using 50µl of buffer and 50µl of 100nM L-364,718 respectively. The assay is terminated by rapid filtration through pre-soaked Whatman GF/B filters using a Brandell Cell Harvester. The filters were washed (3 x 3ml) with ice-cold 50mM Tris HCl (pH 7.4 at 4° ) and bound radioactivity is determined by counting (1 min) in a gamma counter.

The results are set out in Table 3.

**TABLE 3**

| Example | CCK_{A} pKᵢ | Example | CCK_{A} pKᵢ |
|---|---|---|---|
| 1 | 5.7 | 33 | 5.9 |
| 2 | 6.3 | 34 | 6.6 |
| 3 | 5.6 | 35 | 5.9 |
| 4 | 5.9 | 36 | 5.7 |
| 5 | 6.0 | 37 | 5.7 |
| 6 | 5.7 | 38 | 6.5 |
| 7 | 6.3 | 39 | 5.6 |
| 8 | 6.0 | 40 | 6.0 |
| 9 | 6.4 | 41 | 6.0 |
| 10 | 6.1 | 42 | 5.7 |
| 11 | 7.3 | 43 | 5.9 |
| 12 | 5.8 | 45* | 5.9 |
| 13 | 5.7 | 49 | 5.6 |
| 14 | 6.1 | 50 | 6.4 |
| 15 | 7.0 | 51 | 5.8 |
| 16 | 6.0 | 52 | 6.8 |
| 17 | 6.1 | 53 | 5.5 |
| 18 | 6.5 | 54 | 5.8 |
| 19 | 6.4 | 55 | 6.1 |
| 20 | 5.7 | 56 | 6.0 |
| 21 | 5.9 | 61 | 5.6 |
| 22* | 5.9 | 62 | 5.6 |
| 30 | 5.4 | 63 | 5.8 |
| 31 | 5.9 | 64 | 5.8 |
| 32 | 6.3 | | |

| | | | |
|---|---|---|---|
| *- comparative examples | | | |

## Claims

1. A compound of the formula 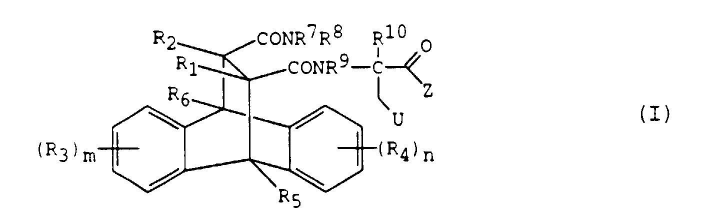 wherein
R¹ and R² are independently H, methyl, halo, carboxy, esterified carboxy, amidated carboxy, carboxymethyl, esterified carboxymethyl or amidated carboxymethyl,
R³ and R⁴ (or each R³ and R⁴ group, when m or n is 2 or more) are independently selected from halo, amino, nitro, cyano, sulphamoyl, sulphonyl, trifluoromethyl, C₁ to C₃ alkyl, C₁ to C₃ alkoxy, hydroxy, C₁ to C₃ hydroxyalkyl, C₁ to C₃ alkylcarboxyamino, carboxy, esterified carboxy and amidated carboxy
R⁵ and R⁶ are independently selected from H and the groups recited above for R³
m is from 0 to 4, provided that m is not more than 2 unless R³ is exclusively halo,
n is from 0 to 4, provided that n is not more than 2 unless R⁴ is exclusively halo,
R⁷' R⁹ and R¹⁰ are independently H or C₁ to C₃ alkyl,
R⁸ is H or C₁ to C₁₅ hydrocarbyl, in which one or more hydrogen atoms of the hydrocarbyl group may be replaced by a halogen atom, and up to two of the carbon atoms may be replaced by a nitrogen, oxygen or sulphur atom, provided that R⁹ does not contain a -O-O-group,
U is aryl, substituted aryl, heterocyclic, substituted heterocyclic or cycloalkyl, and
Z is a group of the formula 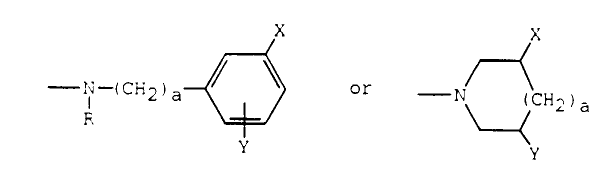
(wherein R is H or C₁ to C₃ alkyl,
X is -CO₂H or tetrazolyl,
Y is H, -CO₂H, tetrazolyl, -CH₂OH, -CO₂Me or -CONH₂, and
a is from 0 to 2)
or a pharmaceutically acceptable salt thereof.

2. A compound according to claim 1, wherein R⁸ is C₆ to C₈ straight or branched chain alkyl or cycloalkyl, or R¹⁰-(CH₂)ₚ-, wherein R¹⁰ is selected from phenyl, 1-naphthyl, 2-naphthyl, indolyl, norbornyl, 1-adamantyl, 2-adamantyl, cyclohexyl or cycloheptyl, and p is from 0 to 3.

3. A compound according to claim 1 or claim 2, wherein R¹ and R² are both H.

4. A compound according to any preceding claim, wherein R⁵ and R⁶ are both H.

5. A compound according to any preceding claim, wherein m and n are both 0.

6. A compound which is degraded *in vivo,* to yield a compound according to any preceding claim.

7. A compound of the formula 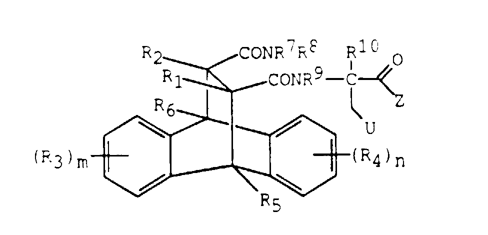 wherein
R¹, R², R³, R⁴, R⁵, R⁶, R⁷, R⁸, R⁹, R¹⁰, m, n and U are as defined in claim 1, and
Z is a group of the formula 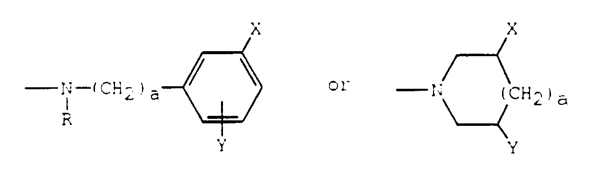 (wherein R is as defined in claim 1; X is esterified carboxy or amidated carboxy, and Y is H, -CO₂H, esterified carboxy, amidated carboxy, tetrazolyl or -CH₂OH, or X is -CO₂H or tetrazolyl, and Y is esterified carboxy or amidated carboxy; and a is from 0 to 2)
or a pharmaceutically acceptable salt thereof.

8. A pharmaceutical composition comprising a compound according to any preceding claim, together with a pharmaceutically acceptable diluent or carrier.

9. A method of making a compound according to any of claims 1 to 5, said method including the step of reacting a compound of the formula 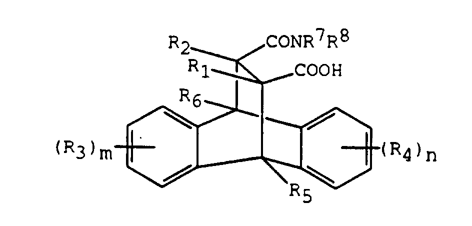 with a suitably protected compound of formula

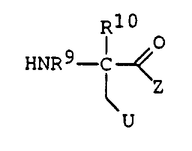

10. A method of making a compound according to any of claims 1 to 5, said method including the step of reacting a compound of the formula 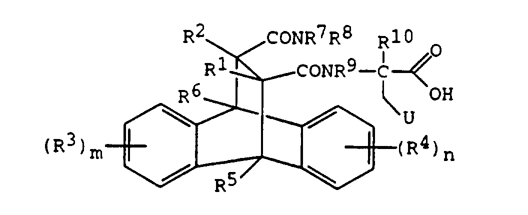 with a suitably protected compound of formula

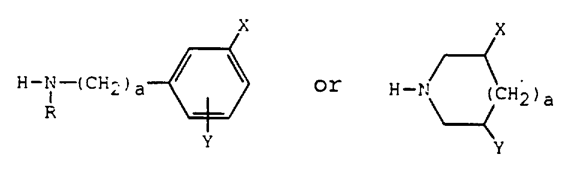

11. A method of making a compound according to any of claims 1 to 5, said method including the step of reacting a compound of the formula 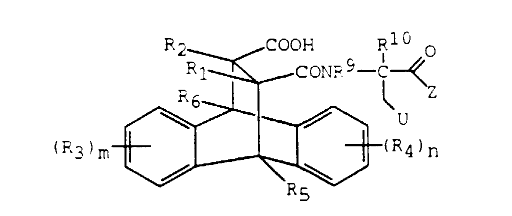 with a suitably protected compound of formula HNR⁷R⁸.

12. A method of making a composition according to claim 8, said method comprising admixing a compound according to any of claims 1 to 5 with a pharmaceutically acceptable diluent or carrier.

## Patentansprüche

1. Verbindung der Formel: 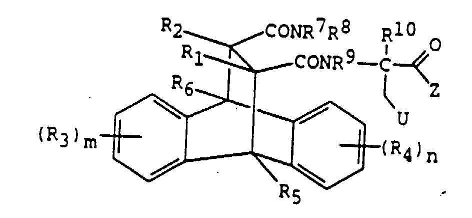 wobei
R¹ und R² unabhängig von einander H, Methyl, Halogen, Carboxyl, verestertes Carboxyl, amidiertes Carboxyl, Carboxymethyl, verestertes Carboxymethyl oder amidiertes Carboxymethyl bedeuten,
R³ und R⁴ (oder jede R³- oder R⁴-Gruppe, wenn m oder n 2 oder mehr beträgt) unabhängig von einander aus Halogen, Amino, Nitro, Zyan, Sulphamyl, Sulphonyl, Trifluormethyl, C₁- bis C₃-Alkyl, C₁- bis C₃-Alkoxyl, Hydroxyl, C₁- bis C₃-Hydroxyalkyl, C₁- bis C₃-Alkylcarboxyamino, Carboxyl, verestertem Carboxyl und amidiertem Carboxyl ausgewählt werden,
R⁵ und R⁶ unabhängig von einander aus H und den oben für R³ genannten Gruppen ausgewählt werden,
m von 0 bis 4 beträgt, vorausgesetzt, daß m nicht mehr als 2 ist, es sei denn R³ ist ausschließlich Halogen,
n von 0 bis 4 beträgt, vorausgesetzt, daß n nicht mehr als 2 ist, es sei denn R⁴ ist ausschließlich Halogen,
R⁷, R⁹ und R¹⁰ unabhängig von einander H oder C₁ bis C₃-Alkyl bedeuten, R⁸ H oder C₁- bis C₁₅-Hydrocarbyl ist, wobei ein oder mehr Wasserstoffatome der Hydrocarbylgruppe durch ein Halogenatom ersetzt werden können und bis zu zwei der Kohlenstoffatome durch ein Stickstoff, Sauerstoff oder Schwefelatom ersetzt werden können, vorausgesetzt, daß R⁹ keine -O-O-Gruppe enthält,
U eine Aryl-, substituierte Aryl-, heterocyclische, substituierte heterocyclische oder Cycloalkyl-Gruppe ist, und
Z eine Gruppe der Formel 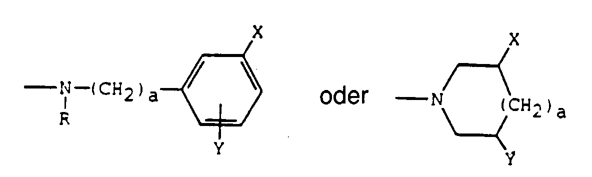
ist
(wobei R H oder C₁- bis C₃-Alkyl ist,
X -CO₂H oder Tetrazolyl ist,
Y H, -CO₂H, Tetrazolyl-, -CH₂OH, - CO₂Me oder -CONH₂ ist und a von 0 bis 2 beträgt).
oder ein pharmazeutisch akzeptierbares Salz hiervon.

2. Verbindung gemäß Anspruch 1, wobei R⁸ eine C₆ bis C₈ gerade oder verzweigte Alkyl- oder Cycloalkyl-Gruppe oder R¹⁰- (CH₂)ₚ- bedeutet, wobei R¹⁰ aus Phenyl, 1-Naphthyl, 2-Naphthyl, Indolyl, Norbornyl, 1-Adamantyl, 2-Adamantyl, Cyclohexyl oder Cycloheptyl ausgewählt wird und p von 0 bis 3 beträgt.

3. Verbindung gemäß Anspruch 1 oder Anspruch 2, wobei R¹ und R² beide H sind.

4. Verbindung gemäß irgendeinem der vorhergehenden Ansprüche, wobei R⁵ und R⁶ beide H sind.

5. Verbindung gemäß irgendeinem der vorhergehenden Ansprüche, wobei m und n beide 0 sind.

6. Verbindung, die *in vivo* zersetzt wird, um eine Verbindung gemäß irgendeinem der vorhergehenden Ansprüche zu liefern.

7. Verbindung der Formel 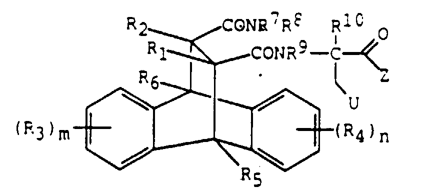 wobei
R¹, R², R³, R⁴, R⁵, R⁶, R⁷, R⁸, R⁹, R¹⁰, m, n und U wie in Anspruch 1 definiert sind und
Z eine Gruppe der Formel 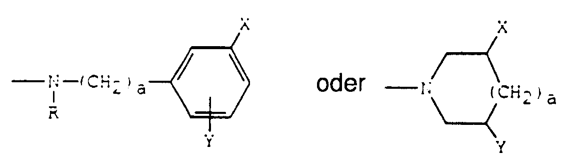
ist
(wobei R wie in Anspruch 1 definiert ist; X verestertes Carboxyl oder amidiertes Carboxyl ist, und Y H, -CO₂H, verestertes Carboxyl, amidiertes Carboxyl, Tetrazolyl oder -CH₂OH ist, oder X -CO₂H oder Tetrazolyl ist und Y verestertes Carboxyl oder amidiertes Carboxyl ist und a von 0 bis 2 beträgt.)
oder ein pharmazeutisch akzeptierbares Salz hiervon.

8. Pharmazeutische Zusammensetzung umfaßend eine Verbindung gemäß irgendeinem der vorhergehenden Ansprüche zusammen mit einem pharmazeutisch akzeptierbaren Verdünner oder Träger.

9. Methode zu Herstellung einer Verbindung gemäß irgendeinem der Ansprüche 1 bis 5, wobei die Methode den Reaktionsschritt einer Verbindung der Formel 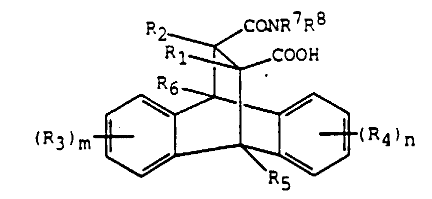 mit einer entsprechend geschützten Verbindung der Formel 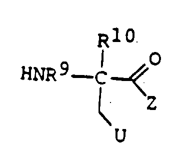 beinhaltet.

10. Methode zu Herstellung einer Verbindung gemäß irgendeinem der Ansprüche 1 bis 5, wobei die Methode den Reaktionsschritt einer Verbindung der Formel 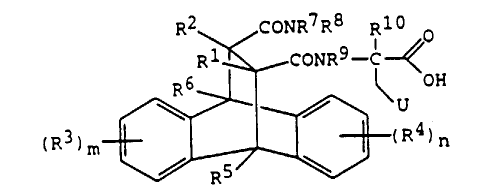 mit einer entsprechend geschützten Verbindung der Formel 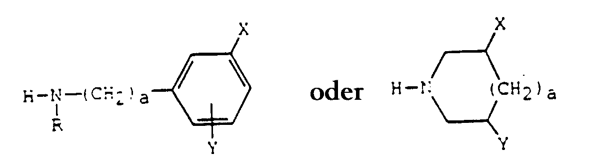 beinhaltet.

11. Methode zu Herstellung einer Verbindung gemäß irgendeinem der Ansprüche 1 bis 5, wobei die Methode den Reaktionsschritt einer Verbindung der Formel 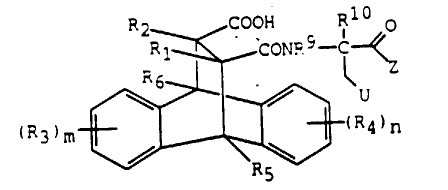 mit einer entsprechend geschützten Verbindung der Formel HNR⁷R⁸
beinhaltet.

12. Methode zur Herstellung einer Zusammensetzung gemäß Anspruch 8, wobei die Methode die Mischung einer Verbindung gemäß irgendeinem der Ansprüche 1 bis 5 mit einem pharmazeutisch akzeptierbaren Verdünner oder Träger umfaßt.

## Revendications

1. Composé répondant à la formule 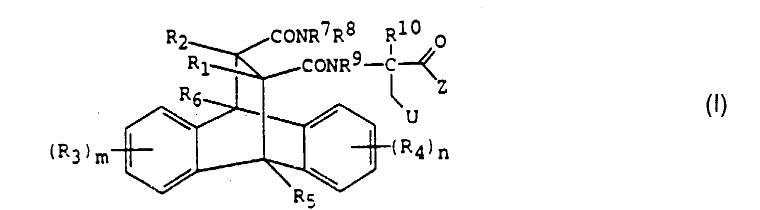 dans laquelle
R¹ et R² représentent indépendamment un atome d'hydrogène, un groupe méthyle, un atome d'halogène, un groupe carboxyle, un groupe carboxyle estérifié, un groupe carboxyle amidé, un groupe carboxyméthyle, un groupe carboxyméthyle estérifié ou un groupe carboxyméthyle amidé,
R³ et R⁴ (ou chaque groupe R³ et R⁴, lorsque m ou n est égal ou supérieur à 2) représentent indépendamment un atome d'halogène, un groupe amino, un groupe nitro, un groupe cyano, un groupe sulfamoyle, un groupe sulfonyle, un groupe trifluorométhyle, un groupe alkyle en C₁-C₃, un groupe alcoxy en C₁-C₃, un groupe hydroxyle, un groupe hydroxyalkyle en C₁-C₃, un groupe (alkyle en C₁-C₃)carboxyamino, un groupe carboxyle, un groupe carboxyle estérifié ou un groupe carboxyle amidé,
R⁵ et R⁶ sont choisis indépendamment parmi un atome d'hydrogène et les groupes cités ci-dessus pour R³,
m vaut de O à 4, à la condition que m ne soit pas supérieur à 2 à moins que R³ représente exclusivement un atome d'halogène,
n vaut de O à 4, à la condition que n ne soit pas supérieur à 2 à moins que R⁴ représente exclusivement un atome d'halogène,
R⁷, R⁹ et R¹⁰ représentent indépendamment un atome d'hydrogène ou un groupe alkyle en C₁-C₃,
R⁸ représente un atome d'hydrogène ou un groupe hydrocarbyle en C₁-C₁₅, où un ou plusieurs atomes d'hydrogène du groupe hydrocarbyle peuvent être remplacés par un atome d'halogène, et jusqu'à deux des atomes de carbone peuvent être remplacés par un atome d'azote, d'oxygène ou de soufre, à la condition que R⁸ ne contienne pas de groupe -O-O-,
U représente un groupe aryle, un groupe aryle substitué, un groupe hétérocyclique, un groupe hétérocyclique substitué ou un groupe cycloalkyle, et
Z représente un groupe répondant à la formule 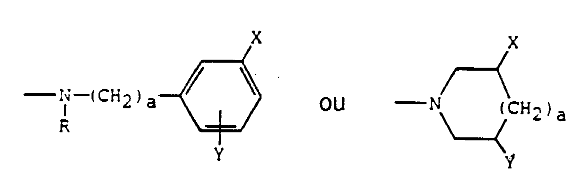
(dans laquelle R représente un atome d'hydrogène ou un groupe alkyle en C₁-C₃,
X représente un groupe -CO₂H ou un groupe tétrazolyle,
Y représente un atome d'hydrogène, un groupe -CO₂H, un groupe tétrazolyle, un groupe -CH₂OH, un groupe -CO₂Me ou un groupe -CONH₂, et
a vaut de 0 à 2),
ou l'un de ses sels pharmaceutiquement acceptables.

2. Composé selon la revendication 1, dans lequel R⁸ représente un groupe alkyle en C₆-C₈ à chaîne linéaire ou ramifiée, ou un groupe cycloalkyle ou un groupe R¹⁰-(CH₂)ₚ, où R¹⁰ est choisi parmi les groupes phényle, 1-naphtyle, 2-naphtyle, indolyle, norbornyle, 1-adamantyle, 2-adamantyle, cyclohexyle ou cycloheptyle, et p vaut de O à 3.

3. Composé selon la revendication 1 ou la revendication 2, dans lequel R¹ et R² représentent tous deux un atome d'hydrogène.

4. Composé selon l'une quelconque des revendications précédentes, dans lequel R⁵ et R⁶ représentent tous deux un atome d'hydrogène.

5. Composé selon l'une quelconque des revendications précédentes, dans lequel m et n sont tous deux égaux à 0.

6. Composé qui est dégradé *in vivo* pour donner un composé selon l'une quelconque des revendications précédentes.

7. Composé répondant à la formule 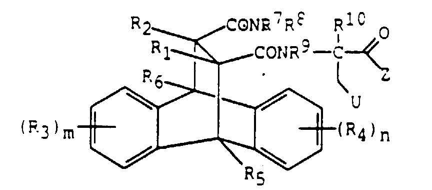 dans laquelle
R¹, R², R³, R⁴, R⁵, R⁶, R⁷, R⁸, R⁹, R¹⁰, m, n et U sont tels que définis dans la revendication 1, et
Z représente un groupe répondant à la formule 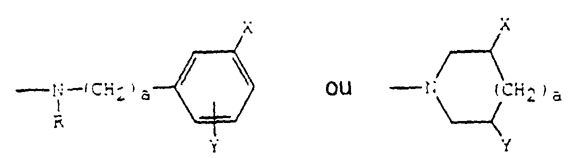
(dans laquelle R est tel que défini dans la revendication 1 ; X représente un groupe carboxyle estérifié ou un groupe carboxyle amidé, et Y représente un atome d'hydrogène, un groupe -CO₂H, un groupe carboxyle estérifié, un groupe carboxyle amidé, un groupe tétrazolyle ou un groupe -CH₂OH ; ou X représente un groupe -CO₂H ou un groupe tétrazolyle, et Y représente un groupe carboxyle estérifié ou un groupe carboxyle amidé ; et a vaut de 0 à 2)
ou l'un de ses sels pharmaceutiquement acceptables.

8. Composition pharmaceutique comprenant un composé selon l'une quelconque des revendications précédentes, ensemble avec un diluant ou un véhicule pharmaceutiquement acceptable.

9. Procédé de préparation d'un composé selon l'une quelconque des revendications 1 à 5, ledit procédé comprenant l'étape de mise en réaction d'un composé répondant à la formule 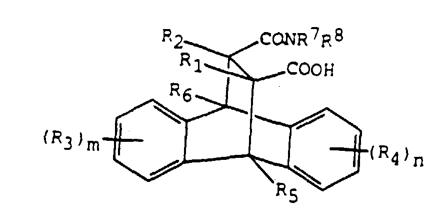 avec un composé convenablement protégé répondant à la formule

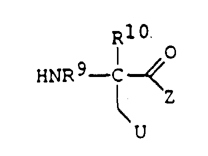

10. Procédé de préparation d'un composé selon l'une quelconque des revendications 1 à 5, ledit procédé comprenant l'étape de mise en réaction d'un composé répondant à la formule 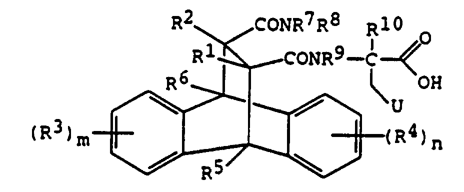 avec un composé convenablement protégé répondant à la formule

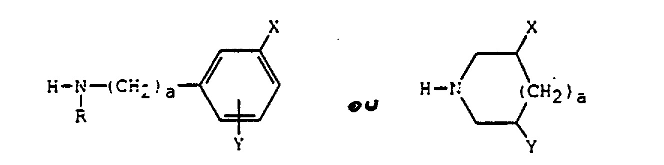

11. Procédé de préparation d'un composé selon l'une quelconque des revendications 1 à 5, ledit procédé comprenant l'étape de mise en réaction d'un composé répondant à la formule 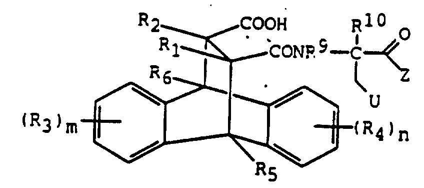 avec un composé convenablement protégé répondant à la formule HNR⁷R⁸.

12. Procédé de préparation d'une composition selon la revendication 8, ledit procédé comprenant le mélange d'un composé selon l'une quelconque des revendications 1 à 5 avec un diluant ou un véhicule pharmaceutiquement acceptable.
